Europäisches Patentamt
European Patent Office
Office européen des brevets

(19) (11) Veröffentlichungsnummer: **0 282 893 A2**

(12) **EUROPÄISCHE PATENTANMELDUNG**

(21) Anmeldenummer: 88103701.4

(22) Anmeldetag: 09.03.88

(51) Int. Cl.⁴: C07D 471/04 , A01N 43/90 , //(C07D471/04,221:00,221:00)

(30) Priorität: 20.03.87 DE 3709263

(43) Veröffentlichungstag der Anmeldung: 21.09.88 Patentblatt 88/38

(84) Benannte Vertragsstaaten: BE CH DE ES FR GB GR IT LI NL

(71) Anmelder: **BAYER AG**
**Konzernverwaltung RP Patentabteilung**
**D-5090 Leverkusen 1 Bayerwerk(DE)**

(72) Erfinder: **Heinemann, Ulrich, Dr.**
**Feldstrasse 18**
**D-5653 Leichlingen 1(DE)**
Erfinder: **Steinbeck, Karl, Dr.**
**12409 Oberbrookroad**
**Leawood 66209 Kansas(US)**
Erfinder: **Berg, Dieter, Dr.**
**Gellertweg 27**
**D-5600 Wuppertal 1(DE)**
Erfinder: **Hänssler, Gerd, Dr.**
**Am Arenzberg 58a**
**D-5090 Leverkusen 3(DE)**
Erfinder: **Reinecke, Paul, Dr.**
**Steinstrasse 8**
**D-5090 Leverkusen 3(DE)**
Erfinder: **Brandes, Wilhelm, Dr.**
**Eichendorffstrasse 3**
**D-5653 Leichlingen 1(DE)**
Erfinder: **Dutzmann, Stefan, Dr.**
**Leinenweberweg 33**
**D-4000 Düsseldorf 31(DE)**

(54) **Schädlingsbekämpfungsmittel auf Basis von 1,10-Phenanthrolinen.**

(57) Die Verwendung von teilweise bekannten 1,10-Phenanthrolinen der Formel (I)

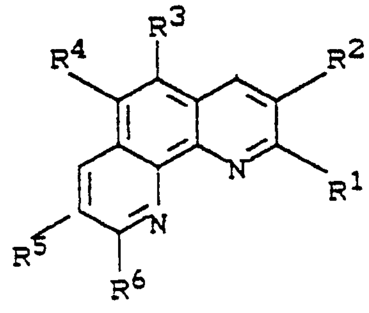

(I)

in welcher

EP 0 282 893 A2

$R^1$ bis $R^6$ die in der Beschreibung gegebenen Bedeutungen haben, zur Bekämpfung von Schädlingen und die noch neuen Verbindungen.

Die bekannten und die neuen 1,10-Phenanthroline der Formel (I) können nach bekannten Verfahren hergestellt werden, z. B. indem man geeignete 8-Aminochinoline mit geeigneten Aldehyden cyclisiert oder geeignete 1,10-Phenanthrolin-Derivate zunächst mit geeigneten elektrophilen Agenzien umsetzt und z. B. Verbindungen mit Nitrogruppen zu Aminogruppen reduziert.

## Schädlingsbekämpfungsmittel auf Basis von 1,10-Phenanthrolinen

Die vorliegende Erfindung betrifft die Verwendung von teilweise bekannten 1,10-Phenanthrolinen zur Bekämpfung von Schädlingen sowie neue 1,10-Phenanthroline und mehrere Verfahren zu ihrer Herstellung.

Es ist bereits bekannt, daß bestimmte organische Stickstoff-Verbindungen, wie beispielsweise das Zink-ethylen-1,2-bis-(dithiocarbamat), fungizide Eigenschaften besitzen (vgl. z.B. R. Wegler "Chemie der Pflanzenschutz-und Schädlingsbekämpfungsmittel", Springer Verlag Berlin, Heidelberg, New York 1970, Band 2, Seite 65f und US-PS 2 457 674).

Darüberhinaus ist bereits bekannt, daß bestimmte Tetrahydrophthalimide, wie beispielsweise cis-N-(-(Trichlormethyl)thio)-4-cyclohexen-1,2-dicarboimid, fungizide Eigenschaften aufweisen (vgl. z.B. Science, (Washington) 115, 84 (1952); US 2 553 770).

Weiterhin ist bereits bekannt, daß bestimmte Perhalogenalkylmercapto-sulfonamide und -sulfamide, wie beispielsweise N,N-Dimethyl-N'-phenyl-N'-(fluordichlormethylthio)-sulfamid, fungizide Eigenschaften aufweisen (vgl. DAS 1 193 498).

Die Wirkung dieser Verbindungen ist jedoch insbesondere bei niedrigen Aufwandmengen und -konzentrationen nicht immer in allen Anwendungsbereichen völlig zufriedenstellend.

Weiterhin sind substituierte 1,10-Phenanthroline bekannt, wie beispielsweise 2-Amino-1,10-phenanthrolin (vgl. Eur. J. Med. Chem. Chim. Ther., 1984-19, 399 - 404), 2-Chlor-o-phenanthrolin (vgl. J. Chem. Soc. 1946, 155 -157) und 2,9-Dichlor-1,10-phenanthrolin (vgl. J. C. S. Perkin I, 1974, 976-978). Über deren Wirksamkeit im Pflanzenschutz ist jedoch nichts bekannt.

Es wurde gefunden, daß die teilweise bekannten substituierten 1,10-Phenanthroline der allgemeinen Formel (I),

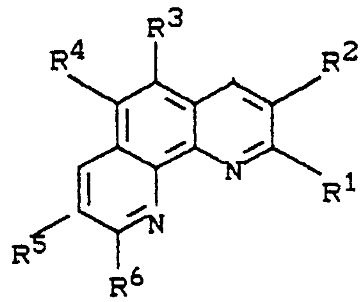

(I).

in welcher

$R^1$ und $R^6$ gleich oder verschieden sind und für Wasserstoff, Alkyl, Halogenalkyl, Halogen, Alkoxy, Alkylthio, Alkylamino oder Dialkylamino stehen,

$R^2$ und $R^5$ gleich oder verschieden sind und für Wasserstoff oder Alkyl stehen,

$R^3$ und $R^4$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, gegebenenfalls substituiertes Aryl, Amino, Alkylamino, gegebenenfalls substituiertes Arylamino, Aralkylamino, Dialkylamino, Diaralkylamino oder für die Gruppierungen -NH-CO-$R^7$, -NH-CY-X-$R^8$, -NH-$SO_2$-$R^9$, -$Y^1$-CO-$R^{10}$, - $Y^2$-CO-$X^1$-$R^{11}$, -$Y^3$-CS-$X^2$-$R^{12}$ oder -CO-$R^{13}$ stehen,

worin

$R^7$, $R^8$, $R^9$, $R^{10}$, $R^{11}$, $R^{12}$ und $R^{13}$ gleich oder verschieden sind und für Alkyl oder gegebenenfalls substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe N-$R^{14}$ stehen, worin $R^{14}$ für Wasserstoff oder Alkyl steht,

sowie deren Säureadditions-Salze und Metallsalz-Komplexe starke biologische Eigenschaften besitzen.

Überraschenderweise zeigen die erfindungsgemäß zu verwendenden substituierten 1,10-Phenanthroline der Formel (I) sowie deren Säureadditions-Salze und Metallsalz-Komplexe eine bessere Wirkung gegen Schädlinge, insbesondere Pilze, als das aus dem Stand der Technik bekannte Zink-ethylen-1,2-bis-(dithiocarbamat), das cis-N-((Trichlormethyl)thio)-4-cyclohexen-1,2-dicarboimid und das N,N-Dimethyl-N'-phenyl-N'-(fluordichlormethylthio)sulfamid.

Die erfindungsgemäß zu verwendenden substituierten 1,10-Phenanthroline stellen somit eine wertvolle

Bereicherung der Technik dar.

Die erfindungsgemäß zu verwendenden substituierten 1,10-Phenanthroline sind durch die Formel (I) allgemein definiert. Bevorzugt werden diejenigen Verbindungen der Formel (I) zur Schädlingsbekämpfung verwendet,

in welcher

$R^1$ und $R^6$ gleich oder verschieden sind und für Wasserstoff, geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Alkoxy oder Alkylthio mit jeweils 1 bis 4 Kohlenstoffatomen, geradkettiges oder verzweigtes Alkylamino oder Dialkylamino mit jeweils 1 bis 4 Kohlenstoffatomen je Alkylrest stehen, und

$R^2$ und $R^5$ gleich oder verschieden sind und für Wasserstoff oder geradkettiges oder verzweigtes Alkyl mit 1 bis 4 Kohlenstoffatomen stehen,

$R^3$ und $R^4$ gleich oder verschieden sind und für Wasserstoff, für geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Hydroxy, Alkoxy oder Alkylthio mit jeweils 1 bis 4 Kohlenstoffatomen, Mercapto, Alkylsulfinyl oder Alkylsulfonyl mit jeweils 1 bis 4 Kohlenstoffatomen, Nitro, gegebenenfalls einfach oder zweifach, gleich oder verschieden durch Halogen, Alkyl oder Halogenalkyl mit jeweils 1 bis 3 Kohlenstoffatomen und 1 bis 5 gleichen oder verschiedenen Halogenatomen substituiertes Phenyl, ferner für Amino, für jeweils geradkettiges oder verzweigtes Alkylamino oder Dialkylamino mit jeweils 1 bis 4 Kohlenstoffatomen je Alkylteil, gegebenenfalls einfach oder zweifach, gleich oder verschieden durch Halogen, Alkyl oder Halogenalkyl mit jeweils 1 bis 3 Kohlenstoffatomen und gegebenenfalls 1 bis 5 gleichen oder verschiedenen Halogenatomen substituiertes Phenylamino, für jeweils gegebenenfalls einfach bis mehrfach, gleich oder verschieden durch Halogen substituiertes Phenylalkylamino oder Diphenylalkylamino mit jeweils 1 oder 2 Kohlenstoffatomen im Alkylteil oder für die Gruppierungen

$-NH-CO-R^7$, $-NH-CY-X-R^8$, $-NH-SO_2-R^9$, $Y^1-CO-R^{10}$, $-Y^2-CO-X^1-R^{11}$, $-Y^3-CS-X^2-R^{12}$ oder $-CO-R^{13}$ stehen, worin

$R^7$, $R^8$, $R^9$, $R^{10}$, $R^{11}$, $R^{12}$ und $R^{13}$ gleich oder verschiedenen sind und für geradkettiges oder verzweigtes Alkyl mit 1 bis 4 Kohlenstoffatomen oder für gegebenenfalls einfach bis dreifach, gleich oder verschieden durch Halogen, Nitro oder Alkyl mit 1 bis 4 Kohlenstoffatomen substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe $N-R^{14}$ stehen, worin $R^{14}$ für Wasserstoff oder geradkettiges oder verzweigtes Alkyl mit 1 bis 4 Kohlenstoffatomen steht.

Bevorzugt erfindungsgemäß zu verwendende Verbindungen sind auch Additionsprodukte aus Säuren und denjenigen substituierten 1,10-Phenanthrolinen der Formel (I) in denen $R^1$, $R^2$, $R^3$, $R^4$, $R^5$ und $R^6$ diejenigen Bedeutungen haben, die im Zusammenhang mit der Beschreibung der erfindungsgemäß zu verwendenden Stoffe bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach bis mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie Imide, wie z.B. Phthalimid, Saccharin und Thiosaccharin.

Außerdem bevorzugt erfindungsgemäß zu verwendende Verbindungen sind auch Additionsprodukte aus Salzen von Metallen der I., II. und III. Hauptgruppe sowie des Zinns, sowie ferner Salze von Metallen der I., II., VII. und VIII. Nebengruppe des Periodensystems der Elemente und denjenigen substituierten 1,10-Phenanthrolinen der Formel (I), in denen $R^1$, $R^2$, $R^3$, $R^4$, $R^5$ und $R^6$ die Bedeutungen haben, die bereits im Zusammenhang mit der Beschreibung der erfindungsgemäß zu verwendenden Stoffe der Formel (I) vorzugsweise für diese Substituenten genannt wurden.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Besonders bevorzugt werden diejenigen Verbindungen der Formel (I) verwendet, in welchen

$R^1$ und $R^6$ gleich oder verschieden sind und für Wasserstoff, Methyl. Ethyl, n-und i-Propyl, n-, i-, s-und t-Butyl, Chlormethyl, Brommethyl, Trichlormethyl, Chlor, Brom, Methoxy, Ethoxy, Methylamino oder Dimethy-

lamino stehen,

$R^2$ und $R^5$ gleich oder verschieden sind und für Wasserstoff, Methyl oder Ethyl stehen,

$R^3$ und $R^4$ gleich oder verschieden sind und für Wasserstoff, Methyl, Ethyl, n-und i-Propyl, Chlor, Brom, Iod, Hydroxy, Methoxy, Ethoxy, n-Propoxy, i-Propoxy, Mercapto, Methylthio, Ethylthio, n-Butylsulfinyl, Methylsulfonyl, Nitro, gegebenenfalls einfach oder zweifach, gleich oder verschieden durch Fluor, Chlor, Brom, Methyl, Ethyl, i-Propyl oder Trifluormethyl substituiertes Phenyl, ferner für Amino, Methylamino, Ethylamino, 2,6-Dichlorbenzylamino, Dimethylamino, Diethylamino, gegebenenfalls einfach oder zweifach, gleich oder verschieden durch Fluor, Chlor, Brom, Methyl, Ethyl, i-Propyl oder Trifluormethyl substituiertes Phenylamino oder für die Gruppierungen

$-NH-CO-R^7$, $-NH-CY-X-R^8$, $-NH-SO_2R^9$, $-Y^1-CO-R^{10}$, $-Y^2-CO-X^1-R^{11}$, $-Y^3-CS-X^2-R^{12}$ oder $-CO-R^{13}$ stehen, worin

$R^7$, $R^8$, $R^9$, $R^{10}$, $R^{11}$, $R^{12}$ und $R^{13}$ gleich oder verschieden sind und für Methyl, Ethyl, n-und i-Propyl, n-, i-, s-und t-Butyl oder für gegebenenfalls einfach oder zweifach, gleich oder verschieden durch Fluor, Chlor, Brom, Nitro, Methyl, Ethyl, Methoxy oder Trifluormethyl substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe $N-R^{14}$ stehen, worin

$R^{14}$ für Wasserstoff oder Methyl steht.

In diesem Zusammenhang sind die gleichen Säureadditions-Salze und Metallsalz-Komplexe zu nennen, die bereits bei der Beschreibung der bevorzugten erfindungsgemäß zu verwendenden substituierten 1,10-Phenanthroline der Formel (I) genannt wurden.

In einzelnen seien außer den bei den Herstellungsbeispielen genannten Verbindungen die folgenden 1,10-Phenanthroline der allgemeinen Formel (I) genannt:

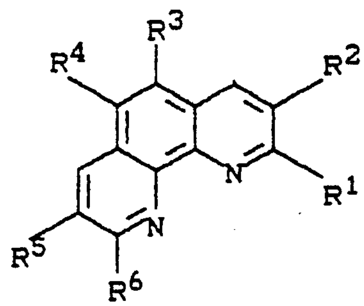

(I)

Tabelle 1:

| $R^1$ | $R^2$ | $R^3$ | $R^4$ | $R^5$ | $R^6$ |
|---|---|---|---|---|---|
| $CH_3$ | H | Br | H | H | $CH_3$ |
| $C_2H_5$ | H | $NO_2$ | H | $CH_3$ | $C_2H_5$ |
| $n-C_3H_7$ | H | H | H | H | $CH_3$ |
| $n-C_3H_7$ | H | Cl | H | H | $C_2H_5$ |
| Cl | H | Br | H | H | Cl |
| $-OCH_3$ | H | $NH_2$ | H | H | H |
| $CH_3NH-$ | H | $CH_3$ | $CH_3$ | H | H |
| $n-C_4H_9$ | H | OH | $CH_3$ | H | $C_2H_5$ |
| $C_2H_5$ | $CH_3$ | Cl | $CH_3$ | H | $CH_3$ |
| $C_2H_5$ | H | -SH | H | H | $C_2H_5$ |
| $n-C_3H_7$ | H | $-OC_2H_5$ | H | H | H |
| $CH_3$ | H | $-SCH_3$ | $CH_3$ | $CH_3$ | $C_2H_5$ |
| $n-C_4H_9$ | H | $CH_3$ | Cl | H | H |
| $CH_3O-$ | H | $-NH_2$ | H | H | $-OCH_3$ |
| Cl | H | J | H | H | Cl |
| Cl | H | $-NHCOCH_3$ | $CH_3$ | H | Cl |
| Cl | $CH_3$ | $CH_3$ | H | $CH_3$ | $C_2H_5$ |
| Cl | H | $C_2H_5$ | $C_2H_5$ | H | $-OCH_3$ |
| $-SCH_3$ | H | H | H | H | $-OC_2H_5$ |
| $-C_4H_9-n$ | H | $-OC_3H_7n$ | H | H | $C_4H_9-n$ |

| $R^1$ | $R^2$ | $R^3$ | $R^4$ | $R^5$ | $R^6$ |
|---|---|---|---|---|---|
| $CH_3$ | H | $-COCH_3$ | H | H | $CH_3$ |
| $CH_3$ | H | $CH_3$ | $-COC_6H_5$ | $CH_3$ | $C_2H_5$ |
| $C_2H_5$ | $CH_3$ | $CH_3$ | $CH_3$ | H | $CH_3$ |
| $-SCH_3$ | H | $-SCH_3$ | Cl | H | H |
| $CH_3$ | H | $-NHCOCH_3$ | H | H | $CH_3$ |
| $-C_3H_7-i$ | H | H | H | H | $CH_3$ |
| $CH_3$ | H | $-NHCOC_6H_5$ | H | H | $CH_3$ |
| $-C_4H_9-t$ | H | $-NHCH_3$ | H | H | $t-C_4H_9-$ |
| $C_2H_5$ | H | $-N(CH_3)_2$ | H | H | $C_2H_5$ |
| $C_2H_5$ | $CH_3$ | -OH | H | $CH_3$ | $C_2H_5$ |
| $CH_3$ | H | $-OC_3H_7i$ | H | H | $CH_3$ |
| $-SCH_3$ | H | Cl | H | H | $-SCH_3$ |
| $-OC_2H_5$ | $CH_3$ | $CH_3$ | Cl | H | $-OC_2H_5$ |
| Cl | H | $NO_2$ | H | H | Cl |
| Br | H | Cl | H | H | Br |
| $ClCH_2-$ | H | $CH_3$ | $CH_3$ | H | H |
| $BrCH_2-$ | H | H | H | H | $i-OCH_3$ |
| $Cl_3C-$ | H | Cl | H | H | $Cl_3C-$ |
| $i-C_4H_9-$ | H | $-NHC_2H_5$ | H | H | $iC_4H_9-$ |
| $CH_3$ | H | $-SOC_4H_9-n$ | $C_2H_5$ | H | $CH_3$ |
| $CH_3$ | H | $-SO_2CH_3$ | H | H | $CH_3$ |
| $CH_3$ | H | $-NHCO-NHCH_3$ | H | H | $CH_3$ |

| $R^1$ | $R^2$ | $R^3$ | $R^4$ | $R^5$ | $R^6$ |
|---|---|---|---|---|---|
| $n-C_4H_9-$ | H | $-NHCO-CHC_6H_5$ | H | H | $CH_3$ |
| $C_2H_5$ | $CH_3$ | $-NH-CS-NHC_6H_5$ | H | H | $CH_3$ |
| $CH_3$ | H | $-NH-CO-OC_2H_5$ | H | H | $CH_3$ |
| $CH_3$ | H | $-N(C_2H_5)$ | H | H | $C_2H_5$ |
| $CH_3$ | H | $-NHCO-SC_3H_7-n$ | H | H | $CH_3$ |
| $CH_3$ | H | $-NHCS-SC_6H_5$ | $CH_3$ | H | $CH_3$ |
| $CH_3$ | H | $-NHCS-OC_4H_9-i$ | $CH_3$ | H | H |
| $C_2H_5$ | H | $-NHSO_2CH_3$ | H | H | $C_2H_5$ |
| $CH_3$ | H | $-OCONHC_6H_5$ | H | H | $CH_3$ |
| $CH_3$ | H | $-OCONHC_3H_7-i$ | H | $C_2H_5$ | $-C_3H_7-n$ |
| $C_2H_5$ | $CH_3$ | $-O-CS-NHC_6H_5$ | $CH_3$ | H | $CH_3$ |
| $n-C_4H_9-$ | H | $-S-CO-OC_2H_5$ | H | H | $-C_4H_9-n$ |
| $n-C_3H_7-$ | $C_2H_5$ | $-S-CS-OC_6H_5$ | $CH_3$ | H | $CH_3$ |
| $CH_3$ | H | $-S-CS-SC_4H_9-n$ | H | $CH_3$ | $C_2H_5$ |
| $CH_3$ | H | $-S-CON(CH_3)_3$ | H | H | $CH_3$ |
| $CH_3$ | H | $-NHC_2H_5$ | H | H | $CH_3$ |
| $CH_3$ | H | $-N(CH_3)_2$ | H | H | $CH_3$ |
| H | H | $-NH_2$ | H | H | H |
| $CH_3$ | H | $-NHCH_2-$ 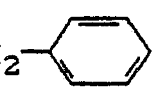 | H | H | $CH_3$ |
| $CH_3$ | H | $-SCH_3$ | H | H | $CH_3$ |

| $R^1$ | $R^2$ | $R^3$ | $R^4$ | $R^5$ | $R^6$ |
|---|---|---|---|---|---|
| H | H | $-SOC_4H_9$-n | H | H | H |
| $CH_3$ | H | $-NHCOC_2H_5$ | H | H | $CH_3$ |
| $C_2H_5$ | H | -NH-CO 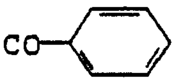 | H | H | $C_2H_5$ |
| $CH_3$ | H | $NO_2$ | $CH_3$ | H | $CH_3$ |
| $CH_3$ | H | $-NH_2$ | $CH_3$ | H | $CH_3$ |
| H | H | $-N(C_2H_5)_2$ | $CH_3$ | H | H |
| $CH_3$ | H | NH 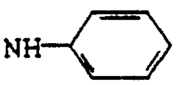 | H | H | $CH_3$ |
| $CH_3$ | H | H | H | H | H |
| $CH_3$ | H | $CH_3$ | H | H | H |
| $CH_3$ | H | $CH_3$ | $CH_3$ | H | $CH_3$ |
| H | H | $CH_3$ | $CH_3$ | H | H |
| $CH_3$ | H | H | $CH_3$ | H | H |
| $CH_3$ | H | $CH_3$ | $CH_3$ | H | H |
| $C_2H_5$ | H | H | H | H | H |
| H | H | $C_2H_5$ | H | H | $CH_3$ |
| n-$C_3H_7$- | H | H | H | H | n-$C_3H_7$- |
| n-$C_4H_9$- | H | H | H | H | $CH_3$ |
| H | H | 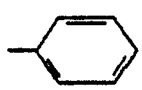 | H | H | H |

| $R^1$ | $R^2$ | $R^3$ | $R^4$ | $R^5$ | $R^6$ |
|---|---|---|---|---|---|
| $CH_3$ | H | 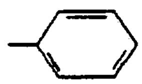 | H | H | $CH_3$ |
| H | H | Cl | H | H | H |
| $CH_3$ | H | Br | H | H | $CH_3$ |
| $n-C_4H_9-$ | H | H | Cl | H | $CH_3$ |
| H | H | $CH_3$ | Br | H | H |
| $CH_3$ | H | $-OCH_3$ | H | H | H |
| $CH_3$ | H | H | $-OC_2H_5$ | H | H |
| $CH_3$ | H | $-OCH_3$ | H | H | $CH_3$ |
| H | H | $-OC_2H_5$ | H | H | H |
| $n-C_3H_7-$ | H | H | $-OCH_3$ | H | $n-C_3H_7-$ |
| H | H | $NO_2$ | H | H | H |
| $CH_3$ | H | H | $NO_2$ | H | H |
| $CH_3$ | H | $-NH_2$ | H | H | $CH_3$ |

Als weitere Beispiele für erfindungsgemäß zu verwendende substituierte 1,10-Phenanthroline der Formel (I) seien die Salze der in Tabelle (I) aufgeführten Verbindungen mit folgenden Säuren genannt: Chlorwasserstoffsäure, Bromwasserstoffsäure, Phosphorsäure, Salpetersäure, Essigsäure, Maleinsäure, Bernsteinsäure, Fumarsäure, Weinsäure, Zitronensäure, Salicylsäure, Sorbinsäure, Milchsäure, Stearinsäure, Ölsäure, Oxalsäure, Malonsäure, Glutarsäure, Ascorbinsäure, Gluconsäure, Phthalimid, Saccharin, Thiosaccharin, Benzol-1,3-disulfonsäure, Benzolsulfonsäure, Naphthalin-2-sulfonsäure, Naphthalin-1,5-disulfonsäure, Methansulfonsäure, gegebenenfalls ein-bis mehrfach durch Nitro oder Chlor substituierte Benzoesäure, Perfluorbutansulfonsäure, Äpfelsäure und Sulfamidsäure.

Als weitere Beispiele für erfindungsgemäß zu verwendende substituierte 1,10-Phenanthroline der Formel (I) seien die Metallsalz-Komplexe mit den in Tabelle (I) aufgeführten Verbindungen genannt.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Die erfindungsgemäß zu verwendenden substituierten 1,10-Phenanthroline der Formel (I) sind teilweise bekannt (vgl. J. Chem. Soc. 1974, 976 - 978; J. Chem. Soc. 1946, 155 - 157; Eur. J. Med. Chem. Chim. Ther., 1984-19, 399-404).

Noch nicht bekannt sind substituierte 1,10-Phenanthroline der Formel (Ia),

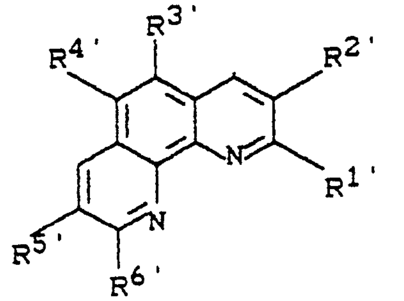

(Ia)

in welcher

$R^{1'}$ und $R^{6'}$ gleich oder verschieden sind und für Wasserstoff, Alkyl, Halogenalkyl, Halogen, Alkoxy, Alkylthio, Alkylamino oder Dialkylamino stehen,

$R^{2'}$ und $R^{5'}$ gleich oder verschieden sind und für Wasserstoff oder Alkyl stehen,

$R^{3'}$ und $R^{4'}$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, gegebenenfalls substituiertes Aryl, Amino, Alkylamino, gegebenenfalls jeweils substituiertes Arylamino oder Aralkylamino, Dialkylamino, gegebenenfalls im Arylteil substituiertes Diaralkylamino oder für die Gruppierungen

$-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen,

worin

$R^7$, $R^8$, $R^9$, $R^{10}$, $R^{11}$, $R^{12}$ und $R^{13}$ gleich oder verschieden sind und für Alkyl oder gegebenenfalls substituiertes Phenyl stehen,

Y, $Y^1$, $Y^2$ und $Y^3$ gleich oder verschieden sind und für Sauerstoff oder Schwefel stehen,

X, $X^1$ und $X^2$ gleich oder verschieden sind und für Sauerstoff, Schwefel oder für die Gruppe $N-R^{14}$ stehen,

worin $R^{14}$ für Wasserstoff oder Alkyl steht,

wobei jedoch a) $R^{1'}$ und $R^{6'}$ nicht gleichzeitig für Wasserstoff stehen oder b) $R^{2'}$, $R^{3'}$, $R^{4'}$ und $R^{5'}$ nicht gleichzeitig für Wasserstoff stehen oder c) für den Fall, daß $R^{3'}$ für Wasserstoff oder Alkyl steht, $R^{1'}$ nicht gleichzeitig für Methyl steht,

sowie deren Säureadditions-Salze und Metallsalz-Komplexe.

Die noch nicht bekannten substituierten 1,10-Phenanthroline sind durch die Formel (Ia) allgemein definiert. Bevorzugt sind diejenigen Verbindungen der Formel (Ia), bei welchen

$R^{1'}$ und $R^{6'}$ gleich oder verschieden sind und für Wasserstoff, geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Alkoxy oder Alkylthio mit jeweils 1 bis 4 Kohlenstoffatomen, geradkettiges oder verzweigtes Alkylamino oder Dialkylamino mit jeweils 1 bis 4 Kohlenstoffatomen je Alkylrest stehen, und

$R^{2'}$ und $R^{5'}$ gleich oder verschieden sind und für Wasserstoff oder geradkettiges oder verzweigtes Alkyl mit 1 bis 4 Kohlenstoffatomen stehen,

$R^{3'}$ und $R^{4'}$ gleich oder verschieden sind und für Wasserstoff, für geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Hydroxy, Alkoxy oder Alkylthio mit jeweils 1 bis 4 Kohlenstoffatomen, Mercapto, Alkylsulfinyl oder Alkylsulfonyl mit jeweils 1 bis 4 Kohlenstoffatomen, Nitro, gegebenenfalls einfach oder zweifach, gleich oder verschieden durch Halogen, Alkyl oder Halogenalkyl mit jeweils 1 bis 3 Kohlenstoffatomen und gegebenenfalls 1 bis 5 gleichen oder verschiedenen Halogenatomen substituiertes Phenyl, ferner für Amino, für jeweils geradkettiges oder verzweigtes Alkylamino oder Dialkylamino mit jeweils 1 bis 4 Kohlenstoffatomen, je Alkylrest, gegebenenfalls einfach oder zweifach, gleich oder verschieden durch Halogen, Alkyl oder Halogenalkyl mit jeweils 1 bis 3 Kohlenstoffatomen und gegebenenfalls 1 bis 5 gleichen oder verschiedenen Halogenatomen substituiertes Phenylamino, für jeweils gegebenenfalls einfach bis mehrfach, gleich oder verschieden durch Halogen substituiertes Phenylalkylamino oder Diphenylalkylamino mit jeweils 1 oder 2 Kohlenstoffatomen im Alkylteil oder für die Gruppierungen

$-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen, worin

$R^7$, $R^8$, $R^9$, $R^{10}$, $R^{11}$, $R^{12}$ und $R^{13}$ gleich oder verschieden sind und für geradkettiges oder verzweigtes Alkyl mit 1 bis 4 Kohlenstoffatomen oder für gegebenenfalls einfach bis dreifach, gleich oder verschieden durch Halogen, Nitro oder Alkyl mit 1 bis 4 Kohlenstoffatomen substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe N-$R^{14}$ stehen, worin $R^{14}$ für Wasserstoff oder geradkettiges oder verzweigtes Alkyl mit 1 bis 4 Kohlenstoffatomen steht,

und wobei jedoch a) $R^{1'}$ und $R^{6'}$ nicht gleichzeitig für Wasserstoff stehen oder b) $R^{2'}$, $R^{3'}$, $R^{4'}$ und $R^{5'}$ nicht gleichzeitig für Wasserstoff stehen, sowie c) für den Fall, daß $R^{3'}$ für Wasserstoff oder Alkyl steht, $R^{1'}$ nich gleichzeitig für Methyl steht.

Bevorzugte erfindungsgemäße Verbindungen sind auch Additionsprodukte aus Säuren und denjenigen substituierten 1,10-Phenanthrolinen der Formel (Ia) in denen $R^{1'}$, $R^{2'}$, $R^{3'}$, $R^{4'}$, $R^{5'}$ und $R^{6'}$ diejenigen Bedeutungen haben, die im Zusammenhang mit der Beschreibung der erfindungsgemäß zu verwendenden Stoffe bereits vorzugsweise für $R^1$, $R^2$, $R^3$, $R^4$, $R^5$ und $R^6$ genannt wurden, ausgenommen die oben angegebenen Verbindungen.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach oder mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie Imide wie z.B. Phthalimid, Saccharin und Thiosaccharin.

Außerdem bevorzugte erfindungsgemäße Verbindungen sind auch Additionsprodukte aus Salzen von Metallen der I., II. und III. Hauptgruppen sowie des Zinns, sowie ferner Salze von Metallen der I., II., VII. und VIII. Nebengruppe des Periodensystems der Elemente und denjenigen substituierten 1,10-Phenanthrolinen der Formel (Ia), in denen $R^{1'}$, $R^{2'}$, $R^{3'}$, $R^{4'}$, $R^{5'}$ und $R^{6'}$ die Bedeutungen haben, die bereits im Zusammenhang mit der Beschreibung der erfindungsgemäß zu verwendenden Stoffe der Formel (I) vorzugsweise für $R^1$, $R^2$, $R^3$, $R^4$, $R^5$ und $R^6$ genannt wurden, ausgenommen die oben genannten Verbindungen.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Besonders bevorzugt sind diejenigen substituierten 1,10-Phenanthroline der Formel (Ia), bei welchen

$R^{1'}$ und $R^{6'}$ gleich oder verschieden sind und für Wasserstoff, Methyl, Ethyl, n-und i-Propyl oder n-, i-, s- und t-Butyl stehen,

$R^{2'}$ und $R^{5'}$ gleich oder verschieden sind und für Wasserstoff, Methyl oder Ethyl stehen,

$R^{3'}$ und $R^{4'}$ gleich oder verschieden sind und für Wasserstoff, Methyl, Chlor, Methoxy, Nitro, Amino, 2,6-Dichlorbenzylamino, Ethylcarbonylamino oder für den Rest

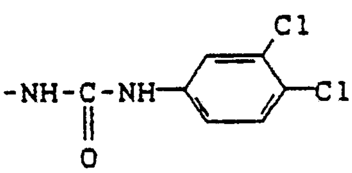

steht,

wobei jedoch a) $R^{1'}$ und $R^{6'}$ nicht gleichzeitig für Wasserstoff stehen oder b) $R^{2'}$, $R^{3'}$, $R^{4'}$ und $R^{5'}$ nicht gleichzeitig für Wasserstoff stehen, sowie c) für den Fall, daß $R^{3'}$ für Wasserstoff oder Alkyl steht, $R^{1'}$ nicht gleichzeitig für Methyl steht.

In diesem Zusammenhang sind die gleichen Säureadditions-Salze und Metallsalz-Komplexe zu nennen, die bereits bei der Beschreibung der bevorzugten erfindungsgemäß substituierten 1,10-Phenanthroline der Formel (Ia) genannt wurden.

Man erhält die substituierten 1,10-Phenanthroline der Formel (Ia), wenn man

A) 8-Aminochinoline der Formel (II),

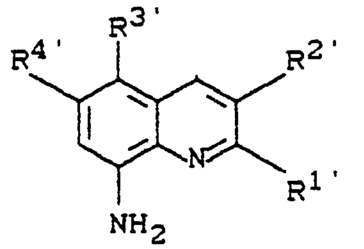

(II)

in welcher

$R^{1'}$, $R^{2'}$, $R^{3'}$ und $R^{4'}$ die oben angegebene Bedeutung haben, wobei jedoch für den Fall, daß $R^{3'}$ für Wasserstoff oder Alkyl steht, $R^{1'}$ nicht gleichzeitig für Methyl steht,

α) mit Aldehyden der Formel (III)

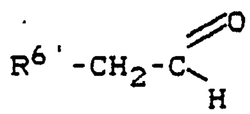

(III)

in welcher

$R^{6'}$ die oben angegebene Bedeutung hat,

gegebenenfalls in Gegenwart von Verdünnungsmitteln sowie gegebenenfalls in Gegenwart starker Säuren cyclisiert und die so erhaltenen Produkte gegebenenfalls in Säureadditions-Salze und Metallsalz-Komplexe überführt, oder

ß) mit α,ß-ungesättigten Aldehyden der Formel (IIIa),

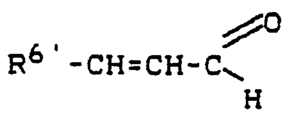

(IIIa)

in welcher

$R^{6'}$ die oben angegebene Bedeutung hat,

gegebenenfalls in Gegenwart von Verdünnungsmitteln sowie gegebenenfalls in Gegenwart einer starken Säure oder gegebenenfalls in Gegenwart eines Oxidationsmittels oder unter Wasserabscheidung gegebenenfalls in Gegenwart eines Katalysators cyclisiert und die so erhaltenen Produkte gegebenenfalls in Säureadditions-Salze und Metallsalz-Komplexe überführt,

oder man erhält die noch nicht bekannten substituierten 1,10-Phenanthroline der Formel (Ia), wenn man

B) substituierte 1,10-Phenanthrolin-Derivate der Formel (Ib),

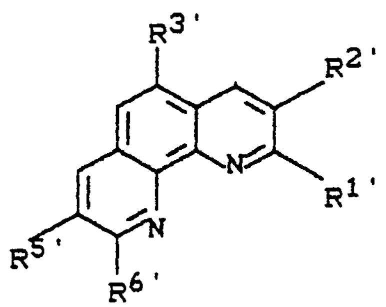

(Ib)

in welcher

$R^{1'}$, $R^{2'}$, $R^{3'}$, $R^{5'}$ und $R^{6'}$ die oben angegebene Bedeutung haben, wobei jedoch a) $R^{1'}$ und $R^{6'}$ nicht gleichzeitig für Wasserstoff stehen oder b) für den Fall, daß $R^{3'}$ für Wasserstoff oder Alkyl steht, $R^{1'}$ nicht gleichzeitig für Methyl steht,

zunächst in einer 1. Stufe in allgemein üblicher Art und Weise mit elektrophilen Agenzien der Formel (IV),

$R^{15}$-E (IV)

in welcher

$R^{15}$ für Halogen, Nitro, Hydroxysulfonyl, Chlorsulfonyl, Alkyl, Formyl, Alkanoyl oder Aroyl steht und

E für eine elektronenanziehende Abgangsgruppierung steht,

oder mit anderen üblichen elektrophilen Reagenzien gegebenenfalls in Gegenwart eines Verdünnungsmittels und gegebenenfalls in Gegenwart eines Katalysators oder Reaktionshilfsmittels in 5- oder 6-Stellung substituiert und diese für den Fall, daß $R^{4'}$ in der Formel (Ia) von einer Nitro-in eine Aminofunktion überführt werden soll, in einer zweiten Stufe gegebenenfalls in Gegenwart von Verdünnungsmitteln und gegebenenfalls in Gegenwart eines Katalysators oder Reaktionshilfsmittels reduziert und gegebenenfalls die so erhaltenen substituierten 1,10-Phenanthroline in deren Säureadditions-Salze oder Metallsalz-Komplexe überführt,

oder man erhält die noch nicht bekannten substituierten 1,10-Phenanthroline der Formel (Ia), wenn man

C) substituierte 1,10-Phenanthrolin-Derivate der Formel (Ic),

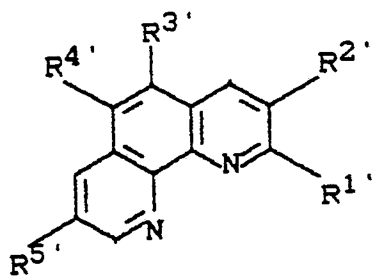

(Ic)

in welcher

$R^{1'}$, $R^{2'}$, $R^{3'}$, $R^{4'}$ und $R^{5'}$ die oben angegebene Bedeutung haben, wobei jedoch a) $R^{2'}$, $R^{3'}$, $R^{4'}$ und $R^{5'}$ nicht gleichzeitig für Wasserstoff stehen oder b) für den Fall, daß $R^{3'}$ für Wasserstoff oder Alkyl steht, $R^{1'}$ nicht gleichzeitig für Methyl steht,

in allgemein üblicher Art und Weise mit Lithiumverbindungen der Formel (V),

$R^{6'}$-Li (V)

in welcher

$R^{6'}$ die oben angegebene Bedeutung hat,

gegebenenfalls in Gegenwart von Verdünnungsmitteln und gegebenenfalls in Gegenwart eines Inertgases umsetzt, anschließend hydrolisiert, danach mit einem Oxidationsmittel dehydriert und die so erhaltenen Produkte gegebenenfalls in deren Säureadditions-Salze oder Metallsalz-Komplexe überführt,

oder

man erhält die substituierten 1,10-Phenanthrolin-Derivate der Formel (Ia), wenn man

D) Verbindungen der Formel (Id),

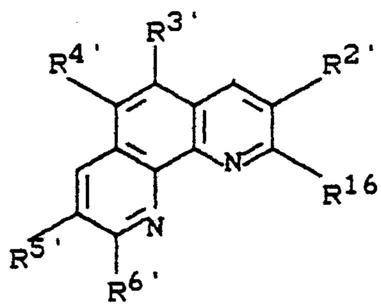

(Id)

in welcher

$R^{16}$ für Halogen steht und

$R^{2'}$, $R^{3'}$, $R^{4'}$, $R^{5'}$ und $R^{6'}$ die oben angegebene Bedeutung haben, wobei $R^{2'}$, $R^{3'}$, $R^{4'}$ und $R^{5'}$ nicht gleichzeitig für Wasserstoff stehen,

gegebenenfalls in Gegenwart von Verdünnungsmitteln und gegebenenfalls in Gegenwart eines Katalysators oder Reaktionshilfsmittels in 2-Stellung durch eine nucleophile Substitution mit Alkoholaten, Thiolaten oder

N,N-Dialkylaminoverbindungen in die entsprechenden 2-Alkyloxy-, 2-Alkylthio-oder 2-N,N-Dialkylamino-1,10-Phenanthroline überführt und die so erhaltenen Produkte gegebenenfalls zu deren Säureadditions-Salzen und Metallsalz-Komplexen weiter umsetzt.

Die bekannten Verbindungen der Formel (I) lassen sich analog den oben genannten Verfahren zur Herstellung der neuen Verbindungen der Formel (Ia) herstellen.

Verwendet man als Ausgangsstoffe beispielsweise 8-Amino-6-chlor-2-methyl-chinolin und Propionaldehyd, so läßt sich der Reaktionsablauf des Herstellungsverfahrens (A/α) durch das folgende Formelschema darstellen:

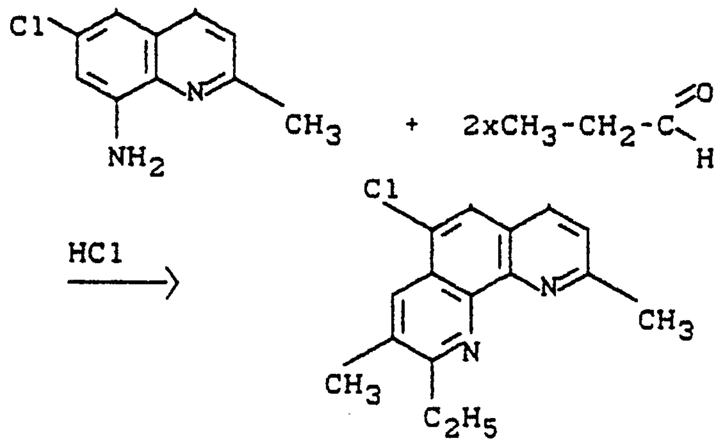


Verwendet man als Ausgangsstoffe beispielsweise 8-Amino-5-chlor-2-methyl-chinolin und Crotonaldehyd, so läßt sich der Reaktionsablauf des Herstellungsverfahrens (A/β) durch das folgende Formelschema darstellen:

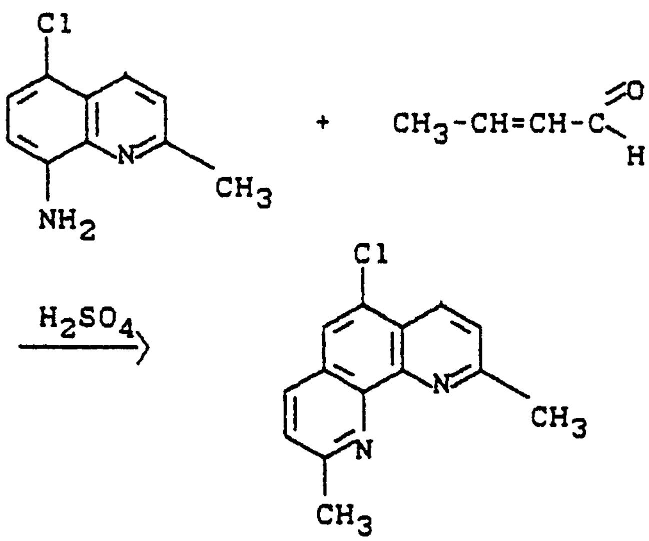


Verwendet man als Ausgangsstoffe beispielsweise 2,9-Dimethyl-1,10-phenanthrolin und in der 1. Stufe Nitriersäure und in der 2. Stufe $SnCl_2$, so läßt sich der Reaktionsablauf des Herstellungsverfahrens (B) durch das folgende Formelschema darstellen:

1. Stufe:

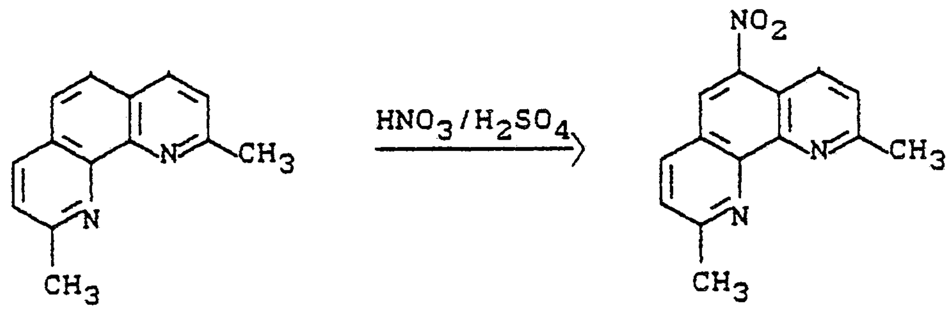


2.Stufe:

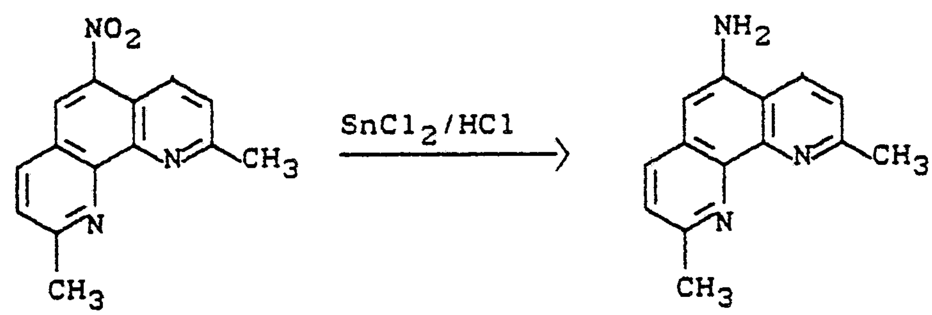


Verwendet man als Ausgangsstoffe beispielsweise 2-Methyl-1,10-phenanthrolin, Butyllithium und Braunstein, so läßt sich der Reaktionsablauf des Herstellungsverfahrens (C) durch das folgende Formelschema darstellen:

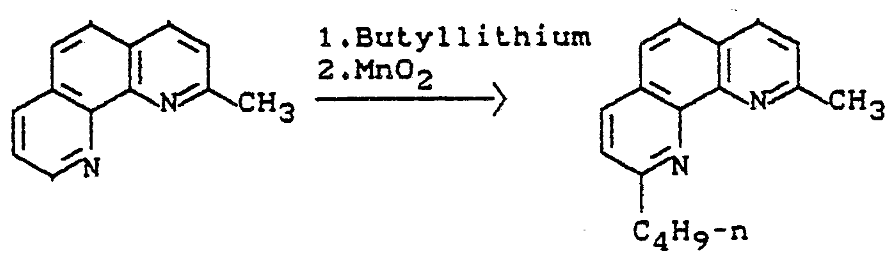


Verwendet man als Ausgangsstoffe beispielsweise 2-Chlor-9-methyl-1,10-phenanthrolin und Natriummethylat, so läßt sich der Reaktionsablauf des Herstellungsverfahrens (D) durch das folgende Formelschema darstellen:

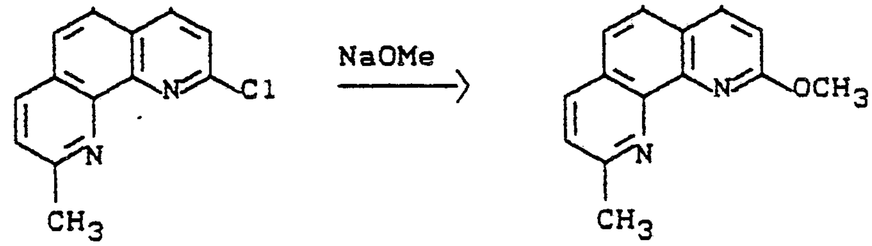


Die zur Durchführung der Herstellungsverfahren (A) als Ausgangsstoffe benötigten 8-Aminochinoline

sind durch die Formel (II) allgemein definiert. In dieser Formel (II) stehen $R^{1'}$, $R^{2'}$, $R^{3'}$ und $R^{4'}$ vorzugsweise bzw. insbesondere für diejenigen Substituenten, die oben bei der Beschreibung der neuen 1,10-Phenanthroline der Formel (Ia) als bevorzugt bzw. besonders bevorzugt für diese Reste genannt wurden.

Die 8-Aminochinoline der Formel (II) sind bekannt und/oder können nach bekannten Verfahren in einfacher, analoger Weise hergestellt werden (vgl. z.B. Org. Reactions 7, 59 ff., (1953); Kirk-Othmer, 2. Aufl., 15, 869), indem man beispielsweise die bekannten 2-Nitroaniline der Formel (VI),

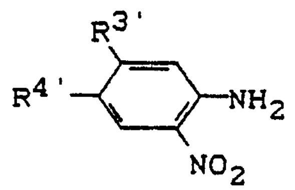

(VI)

in welcher
$R^{3'}$ und $R^{4'}$ die oben angegebene Bedeutung haben,
mit den entsprechenden Aldehyden gegebenenfalls in Gegenwart eines Verdünnungsmittels und gegebenenfalls in Gegenwart eines Katalysators sowie gegebenenfalls in Gegenwart eines Oxidationsmittels, bei Temperaturen zwischen 20 °C und 180 °C zunächst zu den 8-Nitro-chinolinen der Formel (IIa),

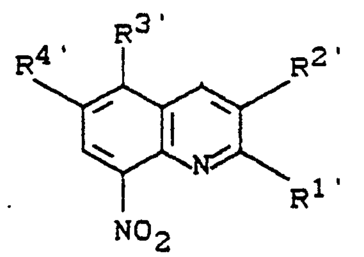

(IIa)

in welcher
$R^{1'}$, $R^{2'}$, $R^{3'}$ und $R^{4'}$ die oben angegebene Bedeutung haben,
cyclisiert und dann die Nitrogruppe gegebenenfalls in Gegenwart eines Verdünnungsmittels in üblicher Art und Weise in Gegenwart eines Katalysators, wie beispielsweise Raney-Nickel, mit Zinn-II-Salzen bei Temperaturen zwischen 20 °C und 150 °C und gegebenenfalls unter Druck zwischen 2 und 100 bar reduziert.

Die weiterhin zur Durchführung der Herstellungsverfahren (A/α) und (A/β) als Ausgangsstoffe benötigten Aldehyde sind durch die Formeln (III) und (IIIa) allgemein definiert. In den Formeln (III) und (IIIa) hat der Rest $R^{6'}$ vorzugsweise bzw. insbesondere diejenige Bedeutung, die oben im Zusammenhang mit der Beschreibung der erfindungsgemäßen Stoffe der Formel (Ia) als bevorzugt bzw. besonders bevorzugt angegeben ist.

Die Verbindungen der Formel (III) und (IIIa) sind allgemein bekannte Verbindungen der organischen Chemie.

Die zur Durchführung des Herstellungsverfahrens (B) als Ausgangsstoffe benötigten substituierten 1,10-Phenanthrolin-Derivate sind durch die Formel (Ib) allgemein definiert. In dieser Formel (Ib) stehen $R^{1'}$, $R^{2'}$, $R^{3'}$, $R^{5'}$ und $R^{6'}$ vorzugsweise bzw. insbesondere für diejenigen Substituenten, die schon bei der Beschreibung der neuen substituierten 1,10-Phenanthroline der Formel (Ia) als bevorzugt bzw. besonders bevorzugt für diese Reste genannt wurden. Die substituierten 1,10-Phenanthrolin-Derivate der Formel (Ib) sind noch nicht bekannt. Man erhält sie nach den Herstellungsverfahren (A/α) oder (A/β) und sie sind Teil der Erfindung.

Die weiterhin zur Durchführung des Herstellungverfahrens (B) als Ausgangsstoffe benötigten elektrophilen Agenzien sind durch die Formel (IV) allgemein definiert. In dieser Formel (IV) steht $R^{14}$ vorzugsweise für Chlor, Brom, Nitroso, Nitro, Hydroxysulfonyl oder Chlorsulfonyl, für Formyl, für geradkettiges oder verzweigtes Alkyl oder Alkanoyl mit jeweils 1 bis 6 Kohlenstoffatomen oder für gegebenenfalls einfach bis mehrfach, gleich oder verschieden substituiertes Benzoyl, wobei als Substituenten infrage kommen: Halogen, insbesondere Fluor, Chlor und Brom, jeweils geradkettiges oder verzweigtes Alkyl, Alkoxy oder Halogenalkyl mit jeweils 1 bis 4 Kohlenstoffatomen und im Fall des Halogenalkyls mit 1 bis 9 gleichen oder verschiedenen Halogenatomen, insbesondere Methyl, Methoxy oder Trifluormethyl.

E steht vorzugsweise für Halogen, insbesondere Chlor oder Brom, für Hydroxy, für Alkyl-oder Arylsulfonyloxy, für Alkanoyloxy oder Aroyloxy. Weiterhin verwendbare elektrophile Reagenzien sind Sulfurylchlorid, Phosphoroxychlorid/Dimethylformamid, Nitriersäure und andere üblicherweise zu elektrophilen Substitutionen verwendeten Stoffe.

Die elektrophilen Agenzien der Formel (IV) ebenso wie die weiteren üblichen elektrophilen Reagenzien sind allgemein bekannte Verbindungen.

.Die zur Durchführung des Herstellungsverfahrens (C) als Ausgangsstoffe benötigten substituierten 1,10-Phenanthrolin-Derivate sind durch die Formel (Ic) allgemein definiert. In dieser Formel (Ic) stehen $R^{1'}$, $R^{2'}$, $R^{3'}$, $R^{4'}$ und $R^{5'}$ vorzugsweise bzw. insbesondere für diejenigen Substituenten, die schon bei der Beschreibung der neuen substituierten 1,10-Phenanthroline der Formel (Ia) als bevorzugt bzw. besonders bevorzugt für diese Reste genannt wurden. Die substituierten 1,10-Phenanthrolin-Derivate der Formel (Ic) sind noch nicht bekannt und sind Teil der Erfindung. Man erhält sie nach den Herstellungsverfahren (A/α) oder (A/β).

Die weiterhin zur Durchführung des Herstellungsverfahrens (C) benötigten Lithiumverbindungen sind durch die Formel (V) allgemein definiert. In dieser Formel (V) steht $R^{6'}$ vorzugsweise für $C_1$-$C_4$-Alkyl. Die Verbindungen der Formel (V) sind allgemein bekannte Verbindungen der organischen Chemie.

Die zur Durchführung des Herstellungsverfahrens (D) als Ausgangsstoffe benötigten substituierten 1,10-Phenanthrolin-Derivate sind durch die Formel (Id) allgemein definiert. In dieser Formel (Id) steht $R^{16}$ für Halogen und $R^{2'}$, $R^{3'}$, $R^{4'}$, $R^{5'}$ und $R^{6'}$ vorzugsweise bzw. insbesondere für diejenigen Substituenten, die - schon bei der Beschreibung der neuen substituierten 1,10-Phenanthroline der Formel (Ia) als bevorzugt bzw. besonders bevorzugt für diese Reste genannt wurden. Die substituierten 1,10-Phenanthrolin-Derivate der Formel (Id) sind noch nicht bekannt und Teil der Erfindung. Man erhält sie nach den Herstellungsverfahren (A/α) oder (A/β).

Die weiterhin zur Durchführung des Herstellungsverfahrens (D) benötigten Alkoholate, Thiolate und N,N-Dialkylamine sind allgemein bekannte Verbindungen der organischen Chemie.

Als Verdünnungsmittel zur Durchführung des Herstellungsverfahrens (A/α) und (A/β) kommen Mischungen von starken anorganischen oder organischen Säuren mit Wasser oder inerten organischen Lösungsmitteln infrage.

Säuren, welche zur Durchführung des Herstellungsverfahrens (A/α) oder A/β) verwendet werden können, sind beispielsweise Schwefelsäure, Salzsäure und p-Toluolsulfonsäure.

Zur Durchführung des Herstellungsverfahrens (A/α) oder (A/β) können praktisch alle inerten organischen Lösungsmittel verwendet werden, insbesondere aromatische, gegebenenfalls halogenierte Kohlenwasserstoffe, wie beispielsweise, Benzin, Benzol, Toluol, Xylol, Chlorbenzol, Dichlorbenzol, Petrolether, Pentan, Hexan, Heptan, Ligroin oder Cyclohexan.

Die Reaktionstemperaturen können bei der Durchführung des Herstellungsverfahrens (A/α) und (A/β) in einem größeren Bereich variiert werden. Im allgemeinen arbeitet man bei Temperaturen zwischen 20 °C und 180 °C, vorzugsweise bei Temperaturen zwischen 50 °C und 130 °C.

Als Oxidationsmittel zur Durchführung des Herstellungsverfahrens (A/β) kommen die für derartige Reaktionen üblichen Oxidationsmittel infrage; vorzugsweise verwendet man Arsensäure oder Nitrogruppen enthaltende aromatische Säuren oder deren Alkalimetall-Salze, wie beispielsweise 3-Nitrobenzolsulfonsäure.

Zur Durchführung der Herstellungsverfahren (A/α) und (A/β) setzt man pro Mol 8-Aminochinolin der Formel (II) 1,0 bis 4,0 Mol, vorzugsweise 1,5 bis 3,5 Mol an Aldehyden der Formel (III) oder (IIIa) und gegebenenfalls 1,0 bis 2,0 Mol, vorzugsweise 1,0 Mol Oxidationsmittel ein. Die Reaktionsführung, Aufarbeitung und Isolierung der Reaktionsprodukte der Formel (I) erfolgt in allgemein üblicher Art und Weise.

Als Verdünnungsmittel zur Durchführung des Herstellungsverfahrens (B) kommen für die erste Reaktionsstufe alle üblicherweise für derartige elektrophile Substitutionen verwendbaren Lösungsmittel infrage. Vorzugsweise verwendet man die als Reagenzien infrage kommenden Säuren oder Gemische, wie beispielsweise Schwefelsäure, Chlorsulfonsäure, Salpetersäure, Nitriersäure, Sulfurylchlorid, Phosphoroxychlorid/Dimethylformamid oder Nitriersäure gleichzeitig als Verdünnungsmittel. Es kommen gegebenenfalls auch inerte organische lösungsmittel, wie beispielsweise Eisessig oder chlorierte Kohlenwasserstoffe, wie Methylenchlorid, Chloroform oder Tetrachlorkohlenstoff als Verdünnungsmittel infrage.

Als Katalysatoren oder Reaktionshilfsmittel zur Durchführung der ersten Reaktionsstufe des Herstellungsverfahrens (B) kommen ebenfalls die für derartige Reaktionen üblichen Katalysatoren infrage; vorzugsweise verwendet man saure Katalysatoren, wie beispielsweise Schwefelsäure, Eisen-III-chlorid oder andere Lewis-Säuren oder Acetanhydrid.

Die Reaktionstemperaturen können bei der Durchführung der ersten Reaktionsstufe des Herstellungsverfahrens (B) in einem größeren Bereich variiert werden. Im allgemeinen arbeitet man zwischen - 50 °C und + 200 °C, vorzugsweise zwischen - 20 °C und + 150 °C.

Zur Durchführung des Herstellungsverfahrens (B) setzt man für die erste Reaktionsstufe pro Mol 1,10-Phenanthrolin der Formel (Ib) im allgemeinen 1,0 bis 150,0 Mol, vorzugsweise 1,0 bis 100 Mol an elektrophilem Agens der Formel (IV) und gegebenenfalls 0,01 bis 30 Mol an Katalysator oder Reaktionshilfsmittel ein. Die Reaktionsführung, Aufarbeitung und Isolierung der Reaktionsprodukte der ersten Reaktionsstufe der Formel (Ib) erfolgt in allgemein üblicher Art und Weise.

Als Verdünnungsmittel zur Durchführung des Herstellungsverfahrens (B) kommen für den Fall, daß $R^{4'}$ von einer Nitro-in eine Aminofunktion überführt werden soll, für die zweite Reaktionsstufe wässrige Systeme, anorganische Säuren oder inerte organische Lösungsmittel infrage. Vorzugsweise verwendet man Wasser, Salzsäure oder ali phatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester oder Sulfoxide, wie Dimethylsulfoxid.

Die Reaktionstemperaturen können bei der Durchführung der zweiten Stufe des Herstellungsverfahrens (B) in einem größeren Bereich variiert werden. Im allgemeinen arbeitet man bei Temperaturen zwischen 20 °C und 150 °C, vorzugsweise bei Temperaturen zwischen 70 °C und 100 °C.

Als Reduktionsmittel zur Durchführung des Herstellungsverfahrens (B) kommen für die zweite Reaktionsstufe für den Fall, das $R^{4'}$ von einer Nitro-in eine Aminofunktion überführt werden soll, alle üblicherweise für derartige Reduktionen verwendbaren Reduktionsmittel infrage. Vorzugsweise verwendet man Zinn-(II)-Salze oder Wasserstoffe/Raney Nickel.

Zur Durchführung des Herstellungsverfahrens (B) setzt man in der zweiten Reaktionsstufe für die Reduktion pro Mol Nitro-1,10-Phenanthrolin im allgemeinen 2,0 bis 6,0 Mol, vorzugsweise 4,0 bis 5,0 Mol Reduktionsmittel ein.

Das Verfahren (B) zur Herstellung der neuen substituierten 1,10-Phenanthroline der Formel (Ia) kann sowohl für die erste als auch für die zweite Reaktionsstufe im allgemeinen bei Normaldruck durchgeführt werden. Unter bestimmten Voraussetzungen kann jedoch auch unter erhöhtem Druck gearbeitet werden.

Die Reaktionsführung, Aufarbeitung und Isolierung der Reaktionsprodukte der Formel (Ib) erfolgt in allgemein üblicher Art und Weise.

Als Verdünnungsmittel zur Durchführung des Herstellungsverfahrens (C) kommen praktisch alle inerten organischen Lösungsmittel infrage. Hierzu gehören vorzugsweise aromatische Kohlenwasserstoffe wie Pentan, Hexan, Heptan, Cyclohexan, Petrolether, Benzin, Ligroin, Benzol, Toluol und Xylol.

Die Reaktionstemperaturen können bei der Durchführung des Herstellungsverfahrens (C) in einem größeren Bereich variiert werden. Im allgemeinen arbeitet man bei Temperaturen zwischen 0 °C und 100 °C, vorzugsweise bei Temperaturen zwischen 25 °C und 50 °C.

Als Inertgase kommen für die Durchführung des Herstellungsverfahrens (C) Stickstoff sowie praktisch alle Edelgase, insbesondere Argon infrage.

Zur Durchführung des Herstellungsverfahrens (C) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt und das Reaktionsgemisch wird mehrere Stunden bei der jeweils erforderlichen Temperatur gerührt. Anschließend wird hydrolisiert und mit einem geeigneten Oxidationsmittel, vorzugsweise Braunstein, dehydriert. Die Aufarbeitung erfolgt jeweils nach üblichen Methoden.

Zur Durchführung des Herstellungsverfahrens (D) kommen als Verdünnungsmittel ebenfalls inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester oder Sulfoxide, wie Dimethylsulfoxid.

Die Reaktionstemperaturen können bei der Durchführung des Herstellungsverfahrens (D) in einem größeren Bereich variiert werden. Im allgemeinen arbeitet man bei Temperaturen zwischen - 50 °C und 200 °C, vorzugsweise bei Temperaturen zwischen - 20 °C und 150 °C. Zur Durchführung des Herstellungsverfahrens (D) werden die jeweils benötigten Ausgangstoffe im allgemeinen in angenähert äquimolaren Mengen eingesetzt. Es ist jedoch auch möglich, eine der beiden jeweils eingesetzten Komponenten in

einem größeren Überschuß zu verwenden. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt jeweils nach üblichen Methoden (vgl. Pijper et. al., Eur. J. Med. Chem. 19, 399, 1984).

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke biologische Wirkung auf und können zur Bekämpfung von unerwünschten Schädlingen praktisch eingesetzt werden. Die Wirkstoffe sind z. B. für den Gebrauch als Pflanzenschutzmittel einsetzbar, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium); Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Kon zentrationen erlaubt eine Behandlung von oberirdischen Pflanzenteilen, von Pflanz-und Saatgut, und des Bodens.

Als Pflanzenschutzmittel können die erfindungsgemäßen Wirkstoffe mit besonders gutem Erfolg protektiv zur Bekämpfung von Erysiphe-Arten an Gerste, Botrytis-Arten bei Bohnen, Pyricularia-Arten an Reis und Venturia-Arten bei Äpfeln oder als Saatgutbehandlungsmittel gegen Drechslera graminea-Arten bei Gerste eingesetzt werden.

Hervorzuheben ist, daß die erfindungsgemäßen Wirkstoffe nicht nur protektiv sondern auch mit besonders gutem Erfolg kurativ zur Bekämpfung von Phytophtora-Arten an Tomaten eingesetzt werden können. Darüberhinaus ist auch die sehr gute systemische Wirkung der organischen Salze der erfindungsgemäßen Wirkstoffe zur Bekämpfung von Pyricularia-Arten an Reis besonders hervorzuheben.

Außerdem besitzen die erfindungsgemäßen Wirkstoffe gegen Rost, Septoria, Cochliobolus sativus und Pyrenophora teres an Getreide sowie gegen Hygiene-und Vorratsschädlinge eine gute Wirkung.

Darüber hinaus zeigen die erfindungsgemäßen Wirkstoffe neben der Leptosphaeria nodorum-Wirkung auch bakterizide Wirkung sowie gute fungizide Wirkung im Myzelwachstumstest.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwen-

dung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den folgenden Beispielen hervor.

Herstellungsbeispiele

Beispiel 1:

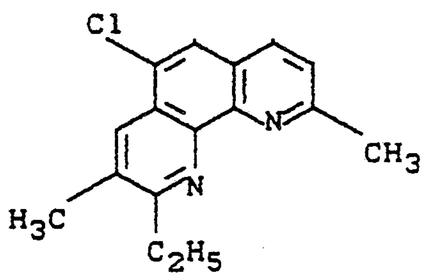

[(I)-1]

(Verfahren A/α)

Zu 19,3 g (0,1 Mol) 8-Amino-6-chloro-2-methylchinolin in 150 ml konzentrierter Salzsäure werden bei 60 °C 11,6 g (0,2 Mol) Propionaldehyd langsam zugetropft, wobei die Temperatur auf 75 °C ansteigt. Nach 1 Stunde Rühren und anschließendem Abkühlen wird die Reaktionsmischung auf Eis gegossen. Der dabei ausfallende Niederschlag wird verworfen. Das Filtrat wird auf pH 5 durch Zugabe von Natronlauge eingestellt, der Niederschlag abfiltriert, in Dichlormethan gelöst und gewaschen. Nach dem Einengen werden 7 g Rohprodukt erhalten. Aus der Mutterlauge wird durch weitere Zugabe von Natronlauge bei pH 11 noch 1 g Rohprodukt isoliert. Nach dem Umkristallisieren der Rohausbeute aus Methanol werden 6 g (22 % der Theorie) 5-Chlor-1-ethyl-2,9-dimethyl-1,10-phenanthrolin vom Schmelzpunkt 171 °C erhalten.

Beispiel 2:

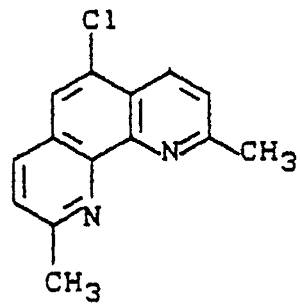

[(I)-2]

(Verfahren A/B)

Zu 20 ml Wasser werden unter Rühren und Eiskühlung 93 ml konzentrierte Schwefelsäure zugetropft und dazu 40,3 g (0,18 Mol) des Natriumsalzes der 3-Nitrobenzolsulfonsäure gegeben. Zu dieser Mischung werden 34,5 g (0,18 Mol) 8-Amino-5-chlor-2-methylchinolin gegeben. In den Ansatz werden bei 105 °C 24,8 ml (0,30 Mol) Crotonaldehyd zugetropft und dann die Mischung 30 Minuten bei 125 °C gerührt. Der Ansatz wird auf 80 °C abgekühlt, auf Wasser gegossen, filtriert und mit Ammoniak-Lösung alkalisch gestellt. Der ausgefallene Niederschlag wird abgesaugt, getrocknet und über eine Kieselgelsäule (Laufmittel Dichlormethan) gereinigt.

Man erhält 4,0 g (9 % der Theorie) 5-Chlor-2,9-dimethyl-1,10-phenanthrolin vom Schmelzpunkt 175 °C - 178 °C.

Beispiel 3:

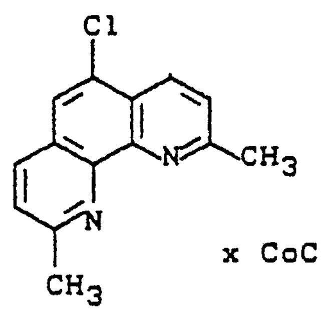

x $CoCl_2$

[(I)-3]

(Verfahren A/B, 2. Stufe)

Zu 3,6 g (0,015 Mol) des nach Beispiel 2 erhaltenen 5-Chlor-2,9-dimethyl-1,10-phenanthrolin in 50 ml Ethanol werden bei Raumtemperatur 3,9 g (0,0165 Mol) $CoCl_2 \times 6H_2O$ in 30 ml Ethanol zugetropft. Nach einer Stunde Rühren bei Raumtemperatur wird der ausgefallene Niederschlag abgesaugt und getrocknet.

Man erhält 5,4 g (96 % der Theorie) des $CoCl_2$-Komplexsalzes des 5-Chlor-2,9-dimethyl-1,10-phenanthrolins mit einem Schmelzpunkt über 240 °C.

Beispiel 4:

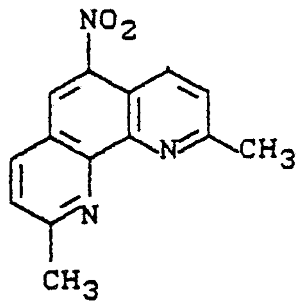

[(I)-4]

(Verfahren B, 1. Stufe)

21 ml (ca. 0,5 Mol) konzentrierte Salpetersäure und 29 ml (ca. 0,5 Mol) konzentrierte Schwefelsäure werden zu Nitriersäure gemischt und diese gekühlt vorgelegt. Nach Zugabe von 10,4 g (0,05 Mol) 2,9-Dimethyl-1,10-phenanthrolin wird 5 Stunden bei 95 °C gerührt. Der Ansatz wird auf Raumtemperatur abgekühlt, in Eiswasser gegossen und mit Natronlauge alkalisch gestellt. Danach wird mit Methylenchlorid extrahiert, mit Wasser gewaschen und zur Trockne eingedampft. Der Rückstand wird in Methylenchlorid gelöst und mit Petrolether erneut ausgefällt. Der Niederschlag wird abgesaugt, mit Petrolether gewaschen und getrocknet.

Man erhält 7,8 g (60 % der Theorie) 2,9-Dimethyl-5-nitro-1,10-phenanthrolin vom Schmelzpunkt 180 °C.

Beispiel 5:

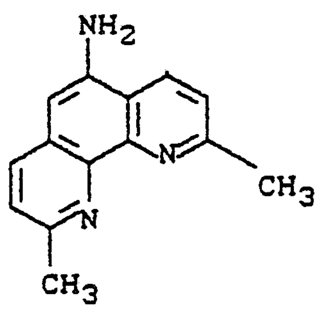

[(I)-5]

(Verfahren B, 2. Stufe)

Zu 27,1 g (0,12 Mol) Zinn-(II)-chlorid-dihydrat in 60 ml konzentrierter Salzsäure werden 7,6 g (0,03 Mol) 2,9-Dimethyl-5-nitro-1,10-phenanthrolin gegeben, und die Mischung bei 90 °C 1 Stunde gerührt. Danach wird der Ansatz auf 0 °C abgekühlt und mit Natronlauge auf pH 13 bis 14 eingestellt. Der gelbe Niederschlag wird isoliert und in viel Dichlormethan aufgenommen. Die Lösung wird mit Wasser gewaschen, die vereinigten organischen Phasen getrocknet und das Lösungsmittel abdestilliert.

Man erhält 3,1 g (48 % der Theorie) 5-Amino-2,9-dimethyl-1,10-phenanthrolin mit einem Schmelzpunkt von über 220 °C.

Beispiel 6:

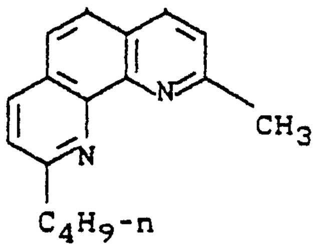

[(I)-6]

(Verfahren C)

3,88 g (0,02 Mol) 2-Methyl-1,10-phenanthrolin werden unter Rühren, Feuchtigkeitsausschluß und Argonspülung in 70 ml wasserfreiem Toluol gelöst und bei Raumtemperatur tropfenweise mit 12,8 ml (0,03 Mol) einer 15prozentigen Lösung von n-Butyllithium in Hexan versetzt und 21 Stunden bei Raumtemperatur nachgerührt. Anschließend wird auf 0 °C abgekühlt und 100 ml Wasser zu dem Ansatz zugetropft. Anschließend wird der Ansatz mit Dichlormethan extrahiert, die vereinigten organischen Phasen mit 200 g (0,475 Mol) Braunstein versetzt und 30 Minuten bei Raumtemperatur gerührt. Danach werden 200 g Natriumsulfat zu dem Reaktionsgemisch gegeben, nach weiteren 30 Minuten Nachrühren wird der Feststoff abgesaugt und das Lösungsmittel abdestilliert. Der verbleibende Rückstand wird über eine Aluminiumoxid-Säule (Laufmittel Methylenchlorid/Cyclohexan 2:1) aufgetrennt.

Man erhält 2,9 g (58 % der Theorie) 2-n-Butyl-9-methyl-1,10-phenanthrolin als braunes Öl.

Beispiel 7:

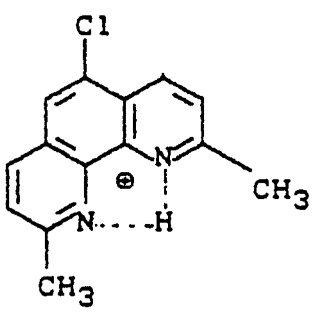

[(I)-7]

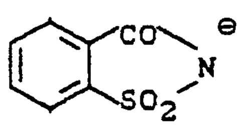

2,1 g (0,0087 Mol) 5-Chlor-2,9-dimethyl-1,10-phenanthrolin und 1,6 g (0,0087 Mol) Saccharin werden in 100 ml Dichlormethan gelöst und 30 Minuten am Rückfluß gekocht. Anschließend wird auf 0 °C abgekühlt und das Lösungsmittel abdestilliert. Der Rückstand wird in Ether aufgenommen und 30 Minuten am Rückfluß gekocht. Nach dem Abkühlen auf Raumtemperatur wird der Feststoff abfiltriert und getrocknet.

Man erhält 3,1 g (84 % der Theorie) des Saccharin-Salzes des 5-Chlor-2,9-dimethyl-1,10-phenanthrolins vom Schmelzpunkt 177 °C - 178 °C.

In analoger Weise zu den in dem Beispielen 1 bis 7 beschriebenen Methoden und unter Berücksichtigung der Angaben in den Beschreibungen zu den erfindungsgemäßen Verfahren werden die nachfolgend aufgeführten Endprodukte der Formel (I),

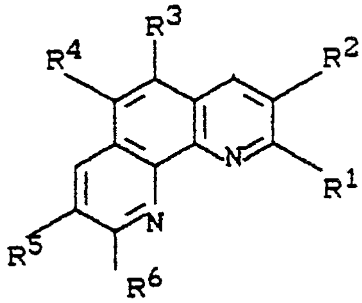

(I)

erhalten:

| Bsp.Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)-8 | $CH_3$ | H | H | Cl | H | $CH_3$ | $CuCl_2 \cdot H_2O$ | 180 |
| (I)-9 | $CH_3$ | H | H | Cl | H | $CH_3$ | $NiCl_2 \cdot H_2O$ | $>240$ |
| (I)-10 | $CH_3$ | H | H | Cl | H | $CH_3$ | $ZnCl_2$ | $>240$ |
| (I)-11 | $CH_3$ | H | H | Cl | H | $CH_3$ | $FeCl_3 \cdot H_2O$ | $>240$ |
| (I)-12 | $CH_3$ | H | $NO_2$ | H | H | $CH_3$ | $NiCl_2 \cdot {}^1/_2H_2O$ | 205 |
| (I)-13 | $CH_3$ | H | $NO_2$ | H | H | $CH_3$ | $FeCl_2 \cdot {}^1/_2H_2O$ | $>220$ |
| (I)-14 | $CH_3$ | H | $NO_2$ | H | H | $CH_3$ | $CuCl_2 \cdot H_2O$ | $>220$ |
| (I)-15 | $CH_3$ | H | $NO_2$ | H | H | $CH_3$ | $CoCl_2$ | $>220$ |
| (I)-16 | $CH_3$ | H | $NO_2$ | H | H | $CH_3$ | $ZnCl_2$ | $>220$ |
| (I)-17 | H | H | H | H | H | H | HCl , $H_2O$ | 215 |
| (I)-18 | $CH_3$ | H | H | Cl | H | H | | 90 |
| (I)-19 | $CH_3$ | H | H | H | H | H | HCl | |
| (I)-20 | H | H | H | $CH_3$ | H | H | | 113 |
| (I)-21 | $CH_3$ | H | H | H | H | $CH_3$ | $CuCl_2$ $CH_3-CH_2-OH$ | |
| (I)-22 | $CH_3$ | H | H | H | H | $CH_3$ | | |

| Bsp. Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)-23 | $CH_3$ | H | H | Cl | H | H | $CuCl_2$, $^1/_2$ $C_2H_5$-OH | 170 |
| (I)-24 | $CH_3$ | H | $CH_3$ | $CH_3$ | H | H | | Öl |
| (I)-25 | $C_2H_5$ | $CH_3$ | H | $-OCH_3$ | H | H | | Öl |
| (I)-26 | $CH_3$ | H | H | $CH_3$ | H | H | | Öl |
| (I)-27 | $CH_3$ | H | H | H | H | $CH_3$ | $MnCl_2$ | |
| (I)-28 | $CH_3$ | H | H | $CH_3$ | H | $CH_3$ | | 70 |
| (I)-29 | $CH_3$ | H | $CH_3$ | $CH_3$ | H | $CH_3$ | | 159 |
| (I)-30 | $nC_4H_9$- | H | H | H | H | $nC_4H_9$- | | 56 |
| (I)-31 | $CH_3$ | H | H | H | H | $CH_3$ | $NiCl_2 \cdot H_2O$ | Zers.$\rangle$300 |
| (I)-32 | $CH_3$ | H | H | H | H | $CH_3$ | $^1/_2CaBr_2 \cdot 3^1/_2H_2O$ | Zers.$\rangle$300 |
| (I)-33 | $CH_3$ | H | H | H | H | $CH_3$ | $^1/_2CaCl_2 \cdot 3^1/_2H_2O$ | Zers.$\rangle$300 |
| (I)-34 | $CH_3$ | H | H | H | H | $CH_3$ | $FeCl_3 \cdot H_2O$ | 172-174 |
| (I)-35 | $CH_3$ | H | H | H | H | $CH_3$ | $SnCl_2 \cdot H_2O$ | 230-235 |
| (I)-36 | $CH_3$ | H | H | H | H | $CH_3$ | $^1/_2MgCl_2 \cdot 3^1/_2H_2O$ | 260 |

| Bsp.Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)-37 | $CH_3$ | H | H | H | H | $CH_3$ | $ZnCl_2$ | 〉300 |
| (I)-38 | $CH_3$ | H | H | H | H | $CH_3$ | $CoCl_2 \cdot {}^1/_2 C_2H_5OH$ | |
| (I)-39 | $CH_3$ | H | H | H | H | $CH_3$ | $FeCl_2 \cdot C_2H_5OH$ | Zers.〉300 |
| (I)-40 | $CH_3$ | H | H | H | H | $CH_3$ | HCl, $H_2O$ | |
| (I)-41 | $CH_3$ | H | H | H | H | H | | 77-78 |
| (I)-42 | $CH_3$ | H | H | H | H | H | $CuCl_2$ | 260-261 |
| (I)-43 | $CH_3$ | H | $NO_2$ | H | H | $CH_3$ | $MnCl_2$ | 295 |
| (I)-44 | $CH_3$ | H | $NH_3^{\oplus}$ | H | H | $CH_3$ | $SnCl_6^{2-} \cdot 2H_2O$ | 262-265 |
| (I)-45 | $CH_3$ | H | $-NH-CH_2-$ 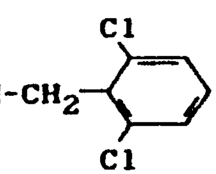 | H | H | $CH_3$ | | 232 Zers. |
| (I)-46 | $CH_3$ | H | $-NH-\underset{\underset{O}{\|}}{C}-CH_2-CH_3$ | H | H | $CH_3$ | | 225-227 |
| (I)-47 | $CH_3$ | H | $-OCH_3$ | H | H | $CH_3$ | | 174-177 |

| Bsp.Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)-48 | $CH_3$ | H | 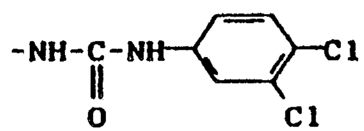 | H | H | $CH_3$ | | 257 |
| (I)-49 | H | H | $NO_2$ | H | H | H | | 198-200 |
| (I)-50 | H | H | $CH_3$ | $CH_3$ | H | H | | 265-266 |
| (I)-51 | H | H | H | $-OCH_3$ | $C_2H_5$ | $CH_3$ | | 82-83 |
| (I)-52 | $CH_3$ | H | H | H | $C_2H_5$ | $CH_3$ | | 100-103 |
| (I)-53 | $CH_3$ | H | H | H | H | $CH_3$ | Saccharat | 186-187 |
| (I)-54 | $CH_3$ | H | $NO_2$ | H | H | $CH_3$ | Saccharat | 195 |
| (I)-55 | $CH_3$ | H | H | H | H | H | Saccharat | 140-141 |
| (I)-56 | H | H | $CH_3$ | H | H | H | Saccharat | 202-203 |
| (I)-57 | $CH_3$ | H | H | H | H | $CH_3$ | Lactat | Öl |
| (I)-58 | $CH_3$ | H | $NH_2$ | H | H | $CH_3$ | Saccharat | 210-213 |
| (I)-59 | $CH_3$ | H | H | H | H | $CH_3$ | Stearat | 85-86 |
| (I)-60 | $CH_3$ | H | H | H | H | $CH_3$ | Succinat | 159-162 |
| (I)-61 | $CH_3$ | H | H | H | H | $CH_3$ | Naphthalin-1,5-disulfonat | $>300$ |

| Bsp.Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)-62 | $CH_3$ | H | $CH_3$ | H | H | $CH_3$ | Saccharat | 165-167 |
| (I)-63 | $CH_3$ | H | H | H | H | $CH_3$ | Maleat | 109-112 |
| (I)-64 | $CH_3$ | H | H | H | H | $CH_3$ | 3,3-Dimethylglutarat | 52-55 |
| (I)-65 | $CH_3$ | H | H | H | H | $CH_3$ | 3,5-Dinitrobenzoat | 202-205 |
| (I)-66 | $CH_3$ | H | H | H | H | $CH_3$ | Perfluorbutansulfonat | 198-200 |
| (I)-67 | $CH_3$ | H | H | H | H | $CH_3$ | CuCl | 328Zers. |
| (I)-68 | $CH_3$ | H | $CH_3$ | H | H | $CH_3$ | $MnCl_2$ x EtOH | 298-299 Zers. |
| (I)-69 | $CH_3$ | H | $CH_3$ | H | H | $CH_3$ | $CuCl_2$ x EtOH | 230Zers. |
| (I)-70 | H | H | Cl | H | H | H | | 123 |
| (I)-71 | H | H | Br | H | H | H | | 80 |
| (I)-72 | $CH_3$ | H | H | Cl | $C_2H_5$ | $CH_3$ | $H_2O$ | 93-95 |
| (I)-73 | $CH_3$ | H | H | H | H | $CH_3$ | Phthalimid | 169-172 |
| (I)-74 | $CH_3$ | H | H | H | H | $CH_3$ | Citrat | 166-167 |
| (I)-75 | $CH_3$ | H | H | H | $CH_3$ | $C_2H_5$ | $H_2O$ | 74-75 |
| (I)-76 | $CH_3$ | H | H | H | $C_2H_5$ | H | | 170-172 |
| (I)-77 | $CH_3$ | H | H | Cl | $CH_3$ | $CH_3$ | | 64-65 |

| Bsp.Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)-78 | $CH_3$ | H | H | H | H | $CH_3$ | 2 × Naphthalin-1,5-disulfonat | 305 Zers. |
| (I)-79 | $CH_3$ | H | H | H | H | $CH_3$ | 1/2 × Naphthalin-1,5-disulfonat × $H_2O$ | 320 |
| (I)-80 | $CH_3$ | H | Cl | H | $CH_3$ | $C_2H_5$ | | 140-41 |
| (I)-81 | $C_2H_5$ | $CH_3$ | $CH_3$ | H | $CH_3$ | $C_2H_5$ | | 118-20 |
| (I)-82 | $C_2H_5$ | $CH_3$ | H | $CH_3$ | $C_2H_5$ | $CH_3$ | | 152-55 |
| (I)-83 | $CH_3$ | H | H | Cl | $CH_3$ | $C_2H_5$ | $CuCl_2 \cdot H_2O$ | 210-14 |
| (I)-84 | $CH_3$ | H | H | Cl | $CH_3$ | $C_2H_5$ | $ZnCl_2$ | 328-30 |
| (I)-85 | $CH_3$ | H | H | Cl | $CH_3$ | $C_2H_5$ | $CoCl_2 \cdot 1/2\ H_2O$ | 326-27 |
| (I)-86 | $CH_3$ | H | H | Cl | $CH_3$ | $C_2H_5$ | Saccharat | 147-48 |
| (I)-87 | $CH_3$ | H | H | Cl | $CH_3$ | $C_2H_5$ | Lactat | 145-49 |
| (I)-88 | $CH_3$ | H | H | Cl | $CH_3$ | $C_2H_5$ | Stearat | 62-64 |
| (I)-89 | $CH_3$ | H | H | Cl | $CH_3$ | $C_2H_5$ | Succinat | 152-55 |
| (I)-90 | $CH_3$ | H | H | H | H | $CH_3$ | Oxalat | 205 |
| (I)-91 | $CH_3$ | H | H | H | H | $CH_3$ | L(+) Tartrat | 178-80 |
| (I)-92 | $CH_3$ | H | H | H | H | $CH_3$ | L(+) Asparaginat | 159-61 |

| Bsp.Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)- 93 | $CH_3$ | H | H | H | H | $CH_3$ | Methansulfonat | 108-10 |
| (I)- 94 | $CH_3$ | H | H | H | H | $CH_3$ | Hexansulfonat | hygroskop. |
| (I)- 95 | $CH_3$ | H | H | H | H | $CH_3$ | Tetradecansulfonat | 130 |
| (I)- 96 | $CH_3$ | H | H | H | H | $CH_3$ | Fumarat | 216-18 |
| (I)- 97 | $CH_3$ | H | H | H | H | $CH_3$ | Malat | 73-90 |
| (I)- 98 | $CH_3$ | H | H | H | H | $CH_3$ | 1/2 Malonat | 170-14 |
| (I)- 99 | $CH_3$ | H | H | H | H | $CH_3$ | 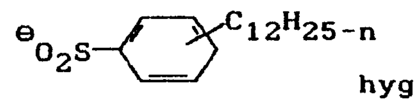 | hygroskop. |
| (I)-100 | $CH_3$ | H | H | H | H | $CH_3$ | Salz der D(-) Chinasäure | 105-108 |
| (I)-101 | $C_2H_5$ | $CH_3$ | Cl | H | $CH_3$ | $C_2H_5$ | | 137-38 |
| (I)-102 | $C_2H_5$ | $CH_3$ | Cl | H | $C_2H_5$ | $CH_3$ | | 104-105 |
| (I)-103 | $C_2H_5$ | $CH_3$ | Cl | H | $CH_3$ | $CH_3$ | | 134-36 |
| (I)-104 | $CH_3$ | H | H | H | $i-C_3H_7$ | $i-C_4H_9$ | | 143-44 |
| (I)-105 | $CH_3$ | H | H | H | $C_2H_5$ | $n-C_3H_7$ | | 102-104 |
| (I)-106 | H | H | Cl | $OCH_3$ | $CH_3$ | $C_2H_5$ | | 165-67 |
| (I)-107 | $CH_3$ | H | H | $OCH_3$ | $CH_3$ | $C_2H_5$ | | 140-43 |

| Bsp.Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)-108 | H | H | H | $OCH_3$ | $CH_3$ | $C_2H_5$ | 2 X $H_2O$ | 70-71 |
| (I)-109 | $CH_3$ | H | H | $OCH_3$ | $CH_3$ | $C_2H_5$ | $CuCl_2$ X 1/2 $H_2O$ | 197-98 |
| (I)-110 | $CH_3$ | H | H | $OCH_3$ | $CH_3$ | $C_2H_5$ | $CoCl_2$ X 1/2 $H_2O$ | 303-304 |
| (I)-111 | $CH_3$ | H | H | $OCH_3$ | $CH_3$ | $C_2H_5$ | Saccharat | 178 |
| (I)-112 | $CH_3$ | H | H | $OCH_3$ | $CH_3$ | $C_2H_5$ | Succinat | 140-42 |
| (I)-113 | $CH_3$ | H | H | $OCH_3$ | $CH_3$ | $C_2H_5$ | Lactat | Öl |
| (I)-114 | $CH_3$ | H | H | $OCH_3$ | $CH_3$ | $C_2H_5$ | $ZnCl_2$ · 1/2 $H_2O$ | 298-300 |
| (I)-115 | $CH_3$ | H | $C(CH_3)_3$ | H | $CH_3$ | $C_2H_5$ | | 131-35 |
| (I)-116 | $CH_3$ | H | H | Cl | $C_2H_5$ | H | | 149-52 |
| (I)-117 | $CH_3$ | H | $SC_2H_5$ | H | $CH_3$ | $C_2H_5$ | | 142-44 |
| (I)-118 | $CH_3$ | H | $SC_2H_5$ | H | H | $CH_3$ | | 144-47 |
| (I)-119 | $CH_3$ | H | H | H | H | $CH_3$ | Salz d. Sorbinsäure | 127-30 |
| (I)-120 | $CH_3$ | H | $SCH_3$ | H | $CH_3$ | $C_2H_5$ | | 115-18 |
| (I)-121 | $CH_3$ | H | $SCH_3$ | H | H | $CH_3$ | | 183-85 |
| (I)-122 | $CH_3$ | H | $SCH_3$ | H | H | $CH_3$ | Saccharat | 177-80 |

| Bsp.Nr. | $R^1$ | $R^2$ | $R^3$ | $R^4$ | $R^5$ | $R^6$ | Metallkomplex, Säureadditions-Salz | Schmelzpunkt °C |
|---|---|---|---|---|---|---|---|---|
| (I)-123 | $CH_3$ | H | $SCH_3$ | H | H | $CH_3$ | | ⟩ 330 |
| (I)-124 | $CH_3$ | H | $SC_2H_5$ | H | $CH_3$ | $C_2H_5$ | | 236-40 |
| (I)-125 | $CH_3$ | H | Cl | H | $CH_3$ | $C_2H_5$ | | 206-208 |
| (I)-126 | $CH_3$ | H | Cl | H | $CH_3$ | $C_2H_5$ | | 266-67 |
| (I)-127 | $CH_3$ | H | Cl | H | $CH_3$ | $C_2H_5$ | 1/3 $Zn_3$ 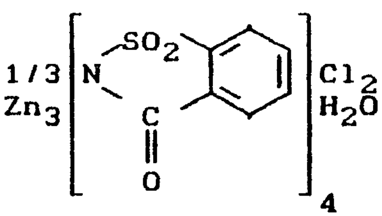 $Cl_2$ · $H_2O$ | 257-6 |
| (I)-128 | $CH_3$ | H | $SCH_3$ | H | $CH_3$ | $C_2H_5$ | $ZnCl_2$ · 1/2 $H_2O$ | 323-26 |
| (I)-129 | $CH_3$ | H | $SCH_3$ | H | $CH_3$ | $C_2H_5$ | Saccharat | 238-40 |
| (I)-130 | $CH_3$ | H | $OCH_3$ | H | H | $CH_3$ | Saccharat | 192-95 |

Herstellung der Ausgangsstoffe

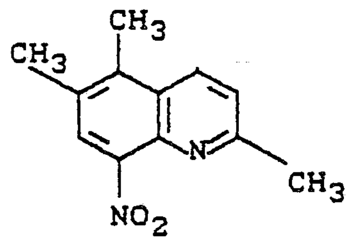

(IIa-1)

67,0 g (0,4 Mol) 4,5-Dimethyl-2-nitroanilin in 240 ml konzentrierter Salzsäure werden tropfenweise bei einer Anfangstemperatur von 55 °C unter gelegentlicher Kühlung mit 68 ml (1,2 Mol) Acetaldehyd versetzt und der Ansatz nach der Hälfte der zugesetzten Menge Acetaldehyd langsam auf 68 °C erwärmt. Nach erfolgter Zugabe wird eine Stunde bei 68 °C weiter erhitzt. Danach wird die Reaktionsmischung auf 40 °C abgekühlt, in 600 ml Eiswasser gegosssen und unter Eiskühlung mit 25prozentiger Ammoniaklösung neutralisiert. Der ausgefallene Feststoff wird abfiltriert und aus Ethanol umkristallisiert.

Man erhält 52,3 g (60 % der Theorie) 2,5,6-Trimethyl-8-nitrochinolin vom Schmelzpunkt 180-181 °C.

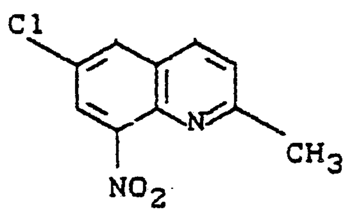

(IIa-2)

Zu 450,0 g (2 Mol) des Natriumsalzes der 3-Nitrobenzolsulfonsäure in 200 ml Wasser werden 1032 ml konzentrierte Schwefelsäure und 346,0 g (2 Mol) 4-Chlor-2-nitroanilin zugetropft. Danach wird der Ansatz auf 80 °C erhitzt, und innerhalb einer Stunde werden 275,6 ml (3,3 Mol) Crotonaldehyd zugetropft. Anschließend wird noch eine weiterer Stunde bei 120 °C bis 125 °C erhitzt. Die Aufarbeitung erfolgt analog Herstellungsbeispiel (IIa-1).

Man erhält 280,0 g (62 % der Theorie) 6-Chlor-2-methyl-8-nitrochinolin vom Schmelzpunkt 125 °C.

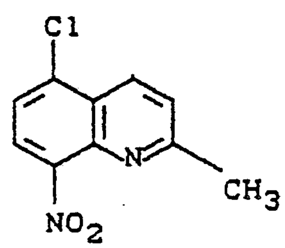

(IIa-3)

17.3 g (0.1 Mol) 5-Chlor-2-nitroanilin und eine Spatelspitze p-Toluolsulfonsäure werden in 200 ml Chlorbenzol auf 110 °C bis 120 °C erhitzt. Zu dieser Mischung werden 14,0 g (0,2 Mol) Crotonaldehyd zugetropft, und der Reaktionsansatz solange bei 120 °C erhitzt, bis die Wasserabscheidung volständig ist. Nach dem Abkühlen auf Raumtemperatur wird das Lösungsmittel im Hochvakuum abdestilliert. Die weitere Aufarbeitung erfolgt analog Herstellungsbeispiel (IIa-1).

Man erhält 8,9 g (45 % der Theorie) 5-Chlor-2-methyl-8-nitrochinolin vom Schmelzpunkt 129 °C.

In analoger Weise zu den in den Beispielen (IIa-1) bis (IIa-3) beschriebenen Methoden werden die nachfolgend aufgeführten Ausgangsstoffe der Formel (IIa),

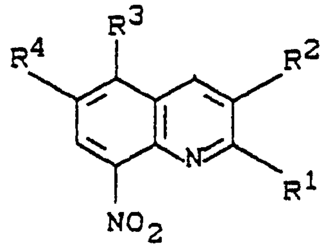

(IIa)

erhalten:

Tabelle 3:

| Bsp. Nr. | $R^1$ | $R^2$ | $R^3$ | $R^4$ | Schmelzpunkt °C |
|---|---|---|---|---|---|
| IIa-4 | $CH_3$ | H | H | $OCH_3$ | 180-183 |
| IIa-5 | $CH_3$ | H | H | $OC_2H_5$ | 139-141 |
| IIa-6 | $CH_3$ | H | H | $CH_3$ | 113 |
| IIa-7 | $C_2H_5$ | $CH_3$ | H | $OCH_3$ | 155 |
| IIa-8 | $C_2H_5$ | $CH_3$ | H | $OC_2H_5$ | 140 |
| IIa-9 | $C_2H_5$ | $CH_3$ | H | $CH_3$ | 125 |
| IIa-10 | $C_2H_5$ | $CH_3$ | $CH_3$ | $CH_3$ | 153 |
| IIa-11 | $CH_3$ | H | $OCH_3$ | $CH_3$ | 98-100 |
| IIa-12 | $C_2H_5$ | $CH_3$ | Cl | H | 150-53 |
| IIa-13 | $C_2H_5$ | $CH_3$ | H | t.-$C_4H_9$ | 76-79 |
| IIa-14 | $CH_3$ | H | H | t.-$C_4H_9$ | 153-54 |
| IIa-15 | $CH_3$ | H | $SC_2H_5$ | H | 126-27 |
| IIa-16 | $CH_3$ | H | $SCH_3$ | H | 145-46 |

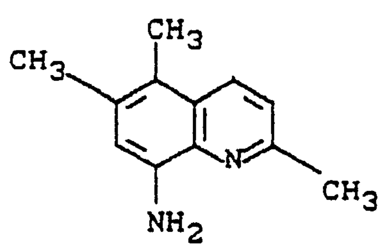

(II-1)

Zu 90,0 g (0,4 Mol) $SnCl_2 \times 2H_2O$ und 180 ml konzentrierter Salzsäure werden 24,0 g (0,1 Mol) 2,5,6-Trimethyl-8-nitrochinolin gegeben und die Mischung 30 Minuten auf 90 °C erhitzt. Danach wird der Ansatz in 500 ml Wasser gegossen und mit Methylenchlorid extrahiert. Die vereinigten organischen Phasen werden gewaschen, mit Natriumsulfat getrocknet und eingeengt.

Man erhält 5,5 g (27 % der Theorie) 8-Amino-2,5,6-trimethyl-chinolin vom Schmelzpunkt 69 °C bis 71 °C.

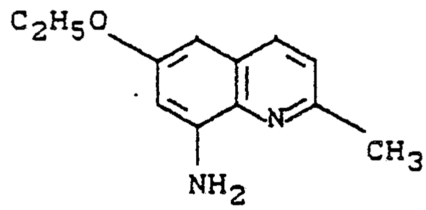

(II-2)

65,3 g (0,28 Mol) 6-Ethoxy-2-methyl-8-nitrochinolin und 10,0 g Raney-Nickel werden in 300 ml Ethanol im Autoklaven 5 Stunden bei 45 °C bis 55 °C und einem Druck von 35 bis 40 bar Wasserstoff hydriert. Danach wird der Katalysator über Kieselgur abfiltriert und das Filtrat eingeengt. Der Rückstand wird in Methylenchlorid aufgenommen, über Kieselgel filtriert und das Filtrat erneut eingeengt.

Man erhält 47,7 g (84 % der Theorie) 8-Amino-6-ethoxy-2-methyl-chinolin vom Schmelzpunkt 69 °C bis 72 °C.

In analoger Weise zu den in den Beispielen (II-1) und (II-2) beschriebenen Methoden werden die nachfolgend aufgeführten Ausgangsstoffe der Formel (II),

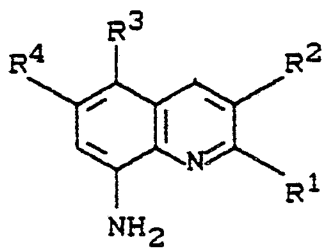

(II)

erhalten:

Tabelle 4:

| Bsp. Nr. | $R^1$ | $R^2$ | $R^3$ | $R^4$ | Schmelzpunkt °C |
|---|---|---|---|---|---|
| II-3 | $CH_3$ | H | H | $OCH_3$ | |
| II-4 | H | H | H | $OCH_3$ | |
| II-5 | $CH_3$ | H | H | $CH_3$ | 100 |
| II-6 | $C_2H_5$ | $CH_3$ | H | $OCH_3$ | 65 |

Tabelle 4:

| Bsp. Nr. | $R^1$ | $R^2$ | $R^3$ | $R^4$ | Schmelzpunkt °C |
|---|---|---|---|---|---|
| II-7 | $C_2H_5$ | $CH_3$ | H | $OC_2H_5$ | 75-80 |
| II-8 | $C_2H_5$ | $CH_3$ | H | $CH_3$ | 88 |
| II-9 | $C_2H_5$ | $CH_3$ | $CH_3$ | $CH_3$ | 67 |
| II-10 | $CH_3$ | H | Cl | H | 93-95 |
| II-11 | $CH_3$ | H | $OCH_3$ | $CH_3$ | 82-84 |
| II-12 | $CH_3$ | H | H | Cl | 100-104 |
| II-13 | $C_2H_5$ | $CH_3$ | Cl | H | 76-77 |
| II-14 | $C_2H_5$ | $CH_3$ | H | t.-$C_4H_9$ | Öl |
| II-15 | $CH_3$ | H | H | t.-$C_4H_9$ | 67-69 |
| II-16 | $CH_3$ | H | SH | H | 155-60 |
| II-17 | H | H | $SC_2H_5$ | H | Öl |
| II-18 | $CH_3$ | H | $SCH_3$ | H | 73-74 |

Anwendungsbeispiele:

In den folgenden Anwendungsbeispielen werden die nachstehend aufgeführten Verbindungen als Vergleichssubstanzen eingesetzt:

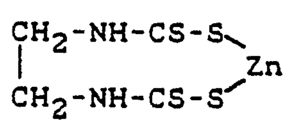

(A)

Zink-ethylen-1,2-bis-(dithiocarbamat)
(bekannt aus R. Wegler "Chemie der Pflanzenschutz-und Schädlingsbekämpfungsmittel", Springer Verlag Berlin, Heidelberg, New York 1970, Band 2, Seite 65 ff; US-PS 2 457 674).

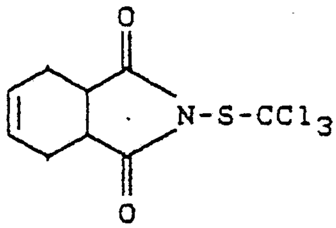

(B)

cis-N-((Trichlormethyl)thio)-4-cyclohexen-1,2-dicarboimid

(bekannt aus Science, (Washington) 115, 84 (1952); US-PS 2 553 770).

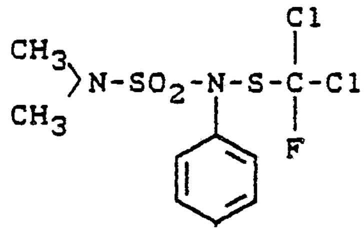

(C)

N,N-Dimethyl-N'-phenyl-N'-(fluordichlormethylthio)sulfamid

(bekannt aus DAS 1 193 498).

Beispiel A

Pyricularia-Test (Reis) /protektiv
Lösungsmittel: 12,5 Gewichtsteile Aceton
Emulgator: 03, Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

In diesem Test zeigen z.B. die erfindungsgemäßen Stoffe [(I)-3], [(I)-5], [(Ia)-7], [(I)-9], [(I)-12]; [(I)-13], [(I)-15], [(I)-20], [(I)-24], [(I)-26], [(I)-27], [(I)-28], [(I)-29], [(I)-30], [(I)-33], [(I)-36], [(I)-38], [(I)-40], [(I)-53], [(I)-54], [(I)-55], [(I)-57], [(I)-59] und [(I)-60] eine bessere Wirksamkeit als die Vergleichsubstanz (A).

Tabelle A

Pyricularia-Test (Reis) /protektiv

| Wirkstoff | Wirkstoffkonzentration in % | Krankheitsbefall in % der unbehandelten Kontrolle |
|---|---|---|
| 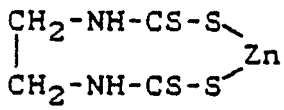 (bekannt) (A) | 0,025 | 75 |
| 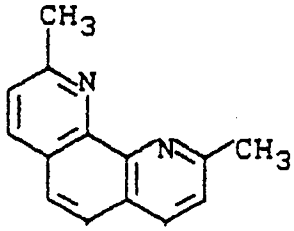 x HCl x $H_2O$ (I)-40 | 0,025 | 0 |
| 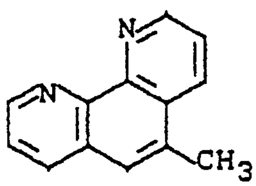 (I)-20 | 0,025 | 0 |
| 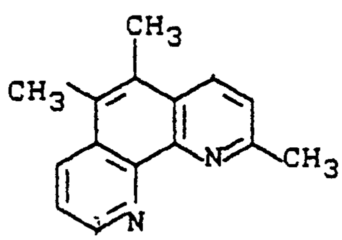 (I)-24 | 0,025 | 0 |

Tabelle A - Fortsetzung

Pyricularia-Test (Reis) /protektiv

| Wirkstoff | Wirkstoffkonzentration in % | Krankheitsbefall in % der unbehandelten Kontrolle |
|---|---|---|
| 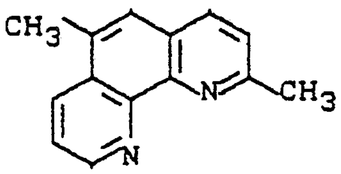 (I)-26) | 0,025 | 0 |
| 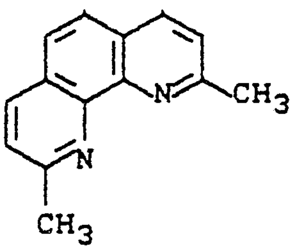 · $MnCl_2$ (I)-27 | 0,025 | 0 |
| 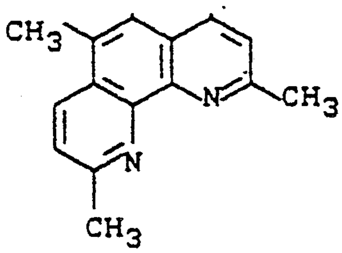 (I)-28 | 0,025 | 0 |

## Tabelle A - Fortsetzung

Pyricularia-Test (Reis) /protektiv

| Wirkstoff | Wirkstoffkonzentration in % | Krankheitsbefall in % der unbehandelten Kontrolle |
|---|---|---|
| 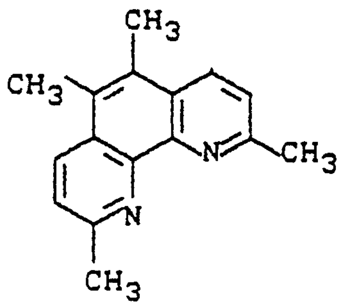 (I)-29 | 0,025 | 0 |
| 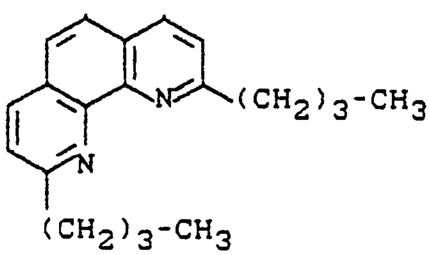 (I)-30 | 0,025 | 0 |
| 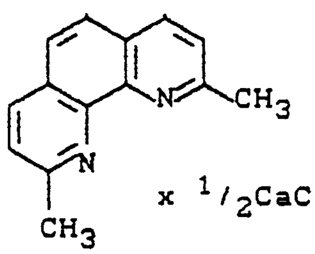 $\times\ ^1/_2CaCl_2 \times 3^1/_2H_2O$ (I)-33 | 0,025 | 0 |

Tabelle A - Fortsetzung

Pyricularia-Test (Reis) /protektiv

| Wirkstoff | Wirkstoffkonzentration in % | Krankheitsbefall in % der unbehandelten Kontrolle |
|---|---|---|
| 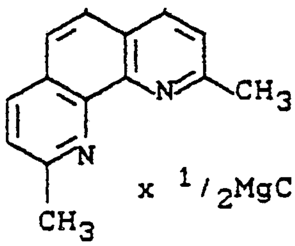 x $^{1}/_{2}MgCl_2$ x $3^{1}/_{2}H_2O$ (I)-36 | 0,025 | 0 |
| 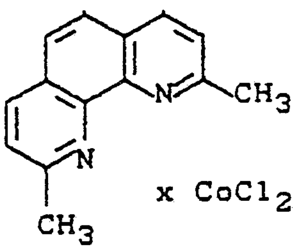 x $CoCl_2$ x $^{1}/_{2}$ EtOH (I)-38 | 0,025 | 0 |
| 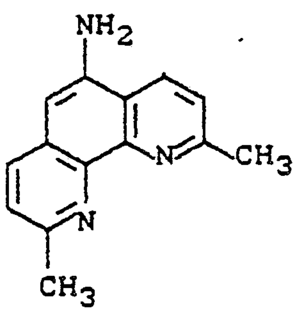 (I)-5 | 0,025 | 0 |

## Tabelle A - Fortsetzung

Pyricularia-Test (Reis) /protektiv

| Wirkstoff | Wirkstoffkonzentration in % | Krankheitsbefall in % der unbehandelten Kontrolle |
|---|---|---|
| 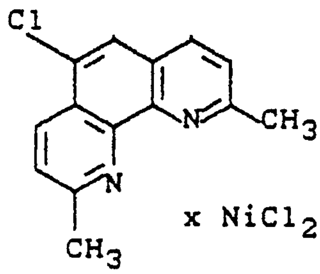 x $NiCl_2$ x $H_2O$ (I)-9 | 0,025 | 0 |
| 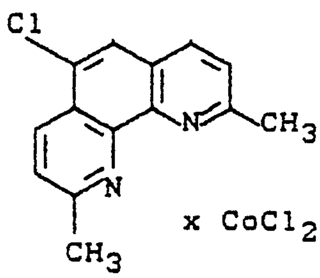 x $CoCl_2$ (I)-3 | 0,025 | 0 |
| 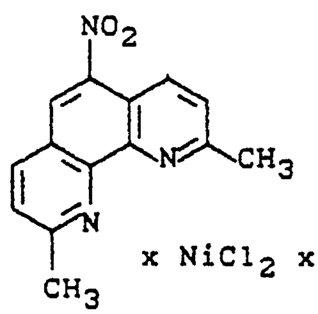 x $NiCl_2$ x $^1/_2H_2O$ (I)-12 | 0,025 | 0 |

Tabelle A - Fortsetzung

Pyricularia-Test (Reis) /protektiv

| Wirkstoff | Wirkstoffkonzentration in % | Krankheitsbefall in % der unbehandelten Kontrolle |
|---|---|---|
| 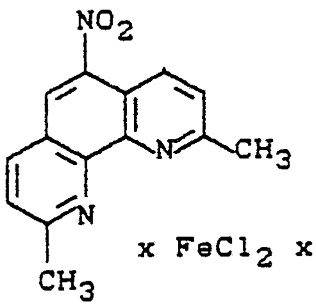 x $FeCl_2$ x $^1/_2H_2O$ (I)-13 | 0,025 | 0 |
| 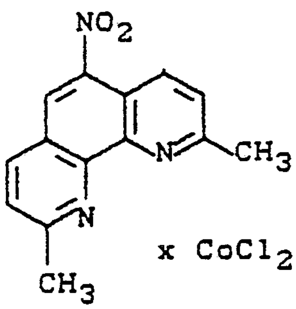 x $CoCl_2$ (I)-15 | 0,025 | 0 |
| 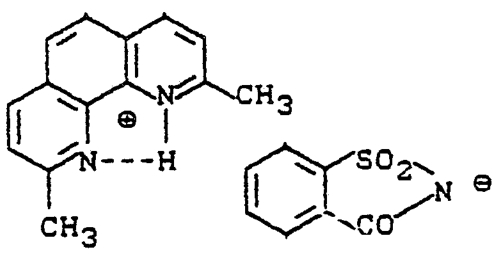 (I)-53 | 0,025 | 0 |

## Tabelle A - Fortsetzung

Pyricularia-Test (Reis) /protektiv

| Wirkstoff | Wirkstoffkonzentration in % | Krankheitsbefall in % der unbehandelten Kontrolle |
|---|---|---|
| 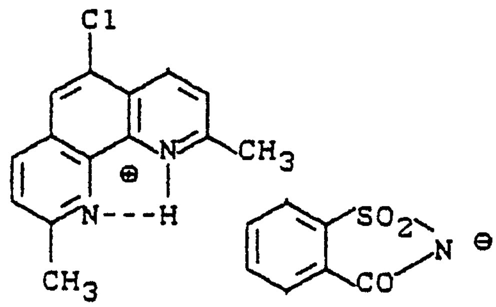 (I)-7 | 0,025 | 0 |
| 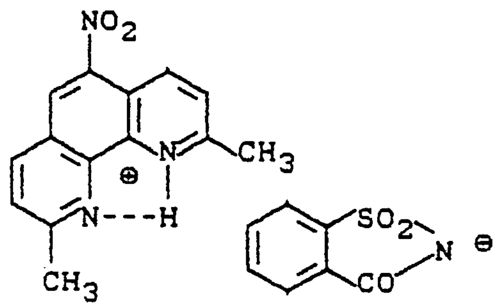 (I)-54 | 0,025 | 0 |
| 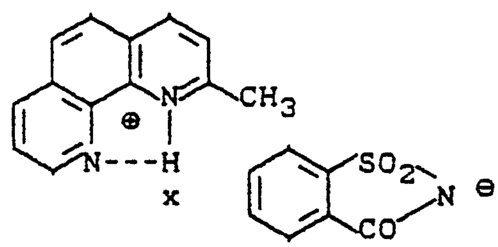 (I)-55 | 0,025 | 0 |

## Tabelle A - Fortsetzung

Pyricularia-Test (Reis) /protektiv

| Wirkstoff | Wirkstoffkonzentration in % | Krankheitsbefall in % der unbehandelten Kontrolle |
|---|---|---|
| 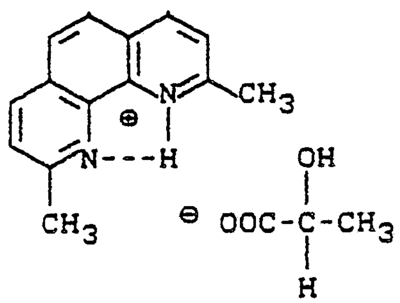 (I)-57 | 0,025 | 0 |
| 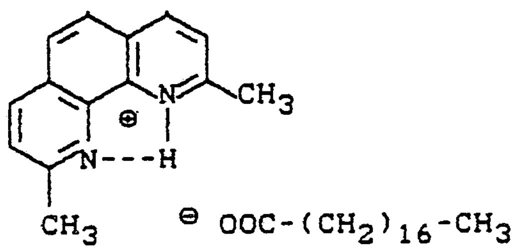 (I)-59 | 0,025 | 0 |
| 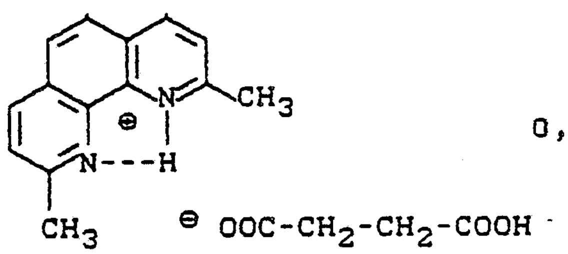 (I)-60 | 0,025 | 0 |

Beispiel B

Pyricularia-Test (Reis) /systemisch Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde

gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

In diesem Test ziegen z.B. die erfindungsgemäßen Stoffe [(I)-7], [(I)-53], [(I)-54], [(I)-55] und [(I)-57] eine bessere Wirksamkeit als die Vergleichsubstanz (A).

## Tabelle B

Pyricularia-Test (Reis) / systemisch

| Wirkstoff | Aufwandmenge in mg Wirkstoff pro 100 $cm^2$ | Krankheitsbefall in % der unbehandelten Kontrolle |
|---|---|---|
| 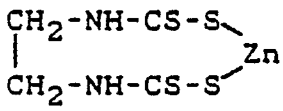 (bekannt) (A) | 100 | 100 |
| 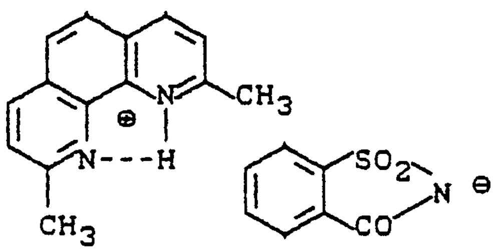 (I)-53 | 100 | 20 |
| 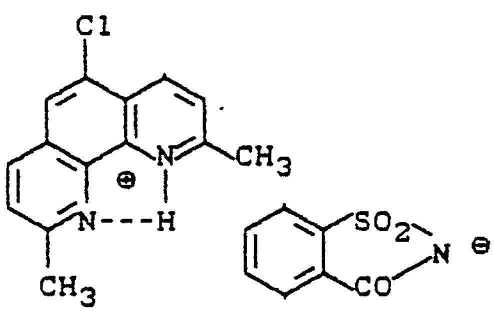 (I)-7 | 100 | 11 |

Tabelle B Fortsetzung

Pyricularia-Test (Reis) / systemisch

| Wirkstoff | Aufwandmenge in mg Wirkstoff pro 100 $cm^2$ | Krankheitsbefall in % der unbehandelten Kontrolle |
|---|---|---|
| 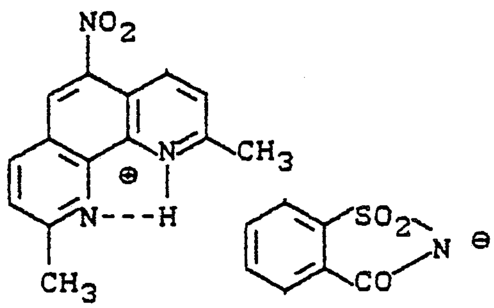 (I)-54 | 100 | 11 |
| 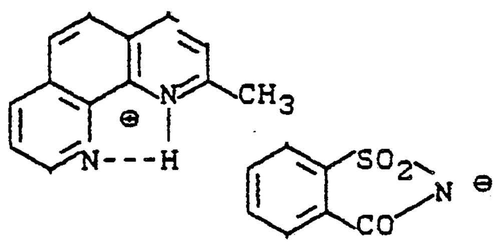 (I)-55 | 100 | 11 |
| 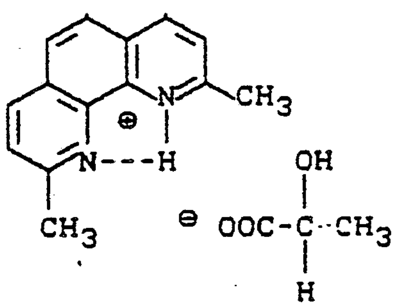 (I)-57 | 100 | 10 |

Beispiel C

Phytophthora-Test (Tomate)/kurativ Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit

den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20°C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100% relativer Luftfeuchtigkeit und ca. 20 °C aufgestellt.

3 Tage nach der Inokulation erfolgt die Auswertung.

In einem Test zeigen z.B. die erfindungsgemäßen Stoffe [(I)-17], [(I)-18], [(I)-20], [(I)-21], [(I)-26], [(I)-29], [(I)-31] und [(I)-33] eine bessere Wirksamkeit als die Vergleichssubstanz (A).

## Tabelle C

Phytophthora-Test (Tomate)/kurativ

| Wirkstoff | Befall in % bei einer Wirkstoffkonzentration von 100 ppm |
|---|---|
| 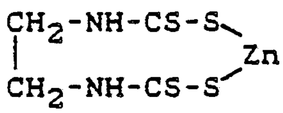 (bekannt) (A) | 41 |
| 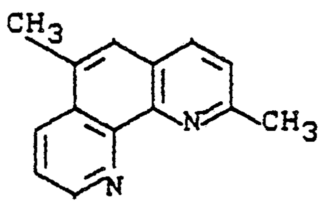 (I)-26 | 10 |
| 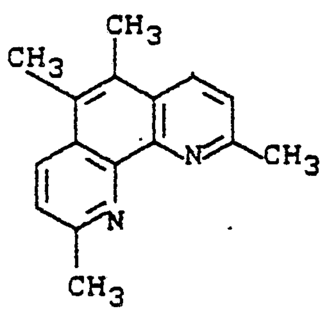 (I)-29 | 15 |
| 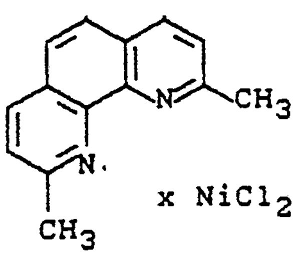 x $NiCl_2$ x $H_2O$ (I)-31 | 10 |

Tabelle C - Fortsetzung

Phytophthora-Test (Tomate)/kurativ

| Wirkstoff | Befall in % bei einer Wirkstoffkonzentration von 100 ppm |
|---|---|
| 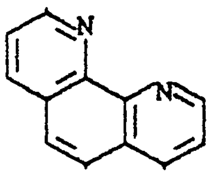 x $H_2O$ x HCl (I)-17 | 15 |
| 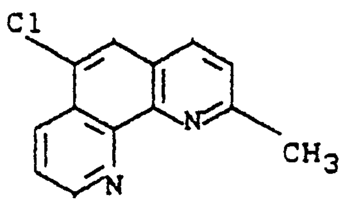 (I)-18 | 18 |
| 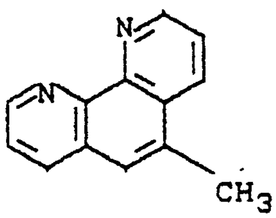 (I)-20 | 10 |
| 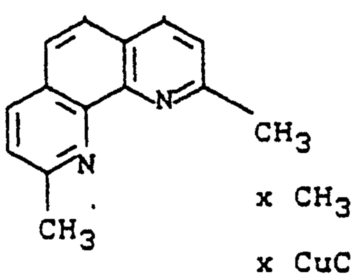 x $CH_3$-$CH_2$-OH x CuCl (I)-21 | 15 |

Tabelle C - Fortsetzung

Phytophthora-Test (Tomate)/kurativ

| Wirkstoff | Befall in % bei einer Wirkstoffkonzentration von 100 ppm |
|---|---|
| 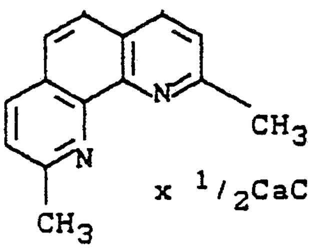 x $^1/_2CaCl_2$ x $3^1/_2$ $H_2O$ (I)-33 | 11 |

Beispiel D

Erysiphe-Test (Gerste) / protektiv Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen z.B. die erfindungsgemäßen Stoffe [(I)-1], [(I)-3], [(I)-4], [(I)-5], [(I)-6], [(I)-7], [(I)-8], [(I)-9], [(I)-10], [(I)-11], [(I)-13], [(I)-14], [(I)-15], [(I)-17], [(I)-18], [(I)-26], [(I)-28], [(I)-30], [(I)-32], [(I)-35], [(I)-39], [(I)-40], [(I)-53], [(I)-55], [(I)-57], [(I)-59] und [(I)-60] eine bessere Wirksamkeit als die Vergleichsubstanz (A).

## Tabelle D

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 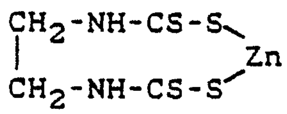 (bekannt) (A) | 0,025 | 100 |
| 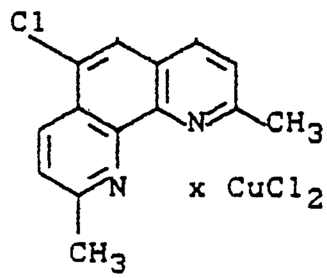 x $CuCl_2$ x $H_2O$ (I)-8 | 0,025 | 9,6 |
| 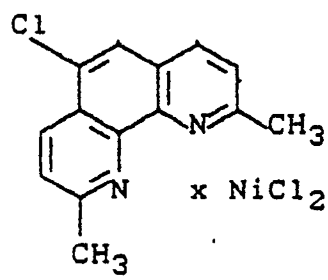 x $NiCl_2$ X $H_2O$ (I)-9 | 0,025 | 13,7 |

Tabelle D - Fortsetzung

Erysiphe-Test (Gerste) / protektiv

| Wirkstoff | Wirkstoff-konzentration in der Spritzbrühe in Gew.-% | Krankheitsbefall in % der unbehandelten Kontrolle |
|---|---|---|
| 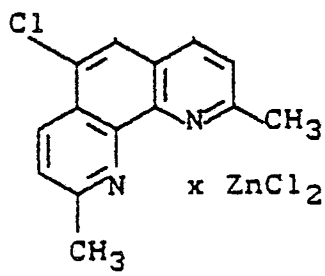 x $ZnCl_2$ (I)-10 | 0,025 | 13,7 |
| 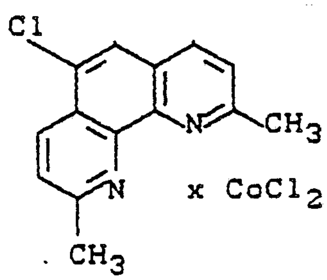 x $CoCl_2$ (I)-3 | 0,025 | 13,7 |
| 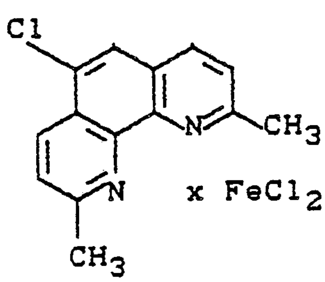 x $FeCl_2$ x $H_2O$ (I)-11 | 0,025 | 13,7 |

Tabelle D - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 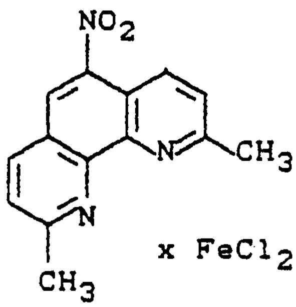 x $FeCl_2$ x $^1/_2H_2O$ (I)-13 | 0,025 | 17,8 |
| 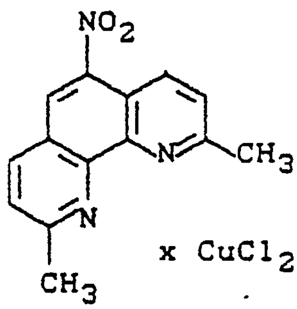 x $CuCl_2$ x $H_2O$ (I)-14 | 0,025 | 45,2 |
| 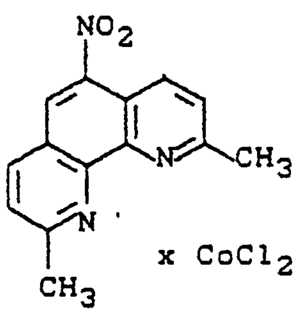 x $CoCl_2$ (I)-15 | 0,025 | 17,4 |

Tabelle D - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 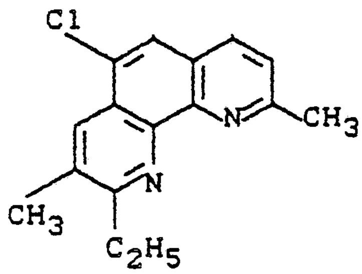 (I)-1 | 0,025 | 12,5 |
| 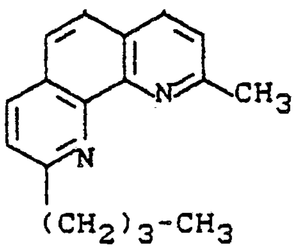 (I)-6 | 0,025 | 0,0 |
| 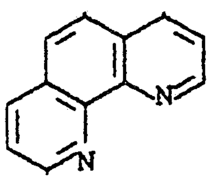 x HCl x $H_2O$ (I)-17 | 0,025 | 12,5 |

Tabelle D - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 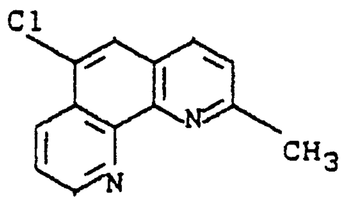 (I)-18 | 0,025 | 0,0 |
| 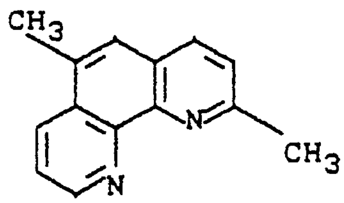 (I)-26 | 0,025 | 12,5 |
| 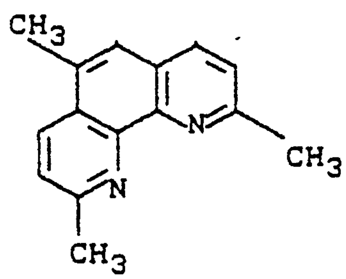 (I)-28 | 0,025 | 0,0 |

Tabelle D - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 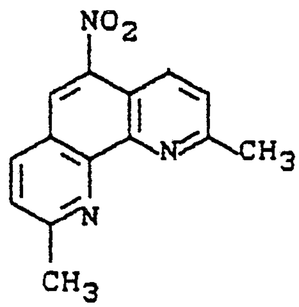 (I)-5 | 0,025 | 49,2 |
| 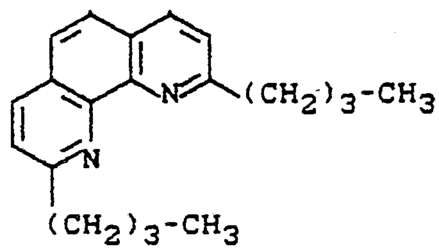 (I)-30 | 0,025 | 12,5 |
| 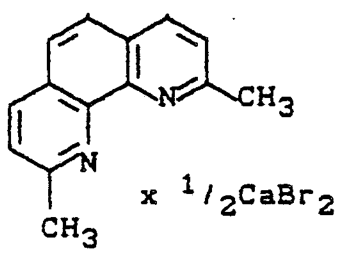 x $^1/_2CaBr_2$ x $3^1/_2H_2O$ (I.)-32 | 0,025 | 0,0 |

Tabelle D - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 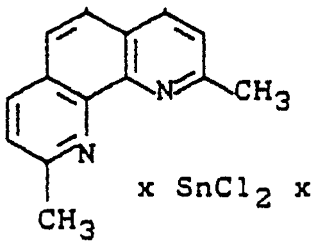 x $SnCl_2$ x $H_2O$ (I)-35 | 0,025 | 3,7 |
| 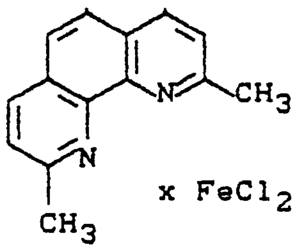 x $FeCl_2$ X EtOH (I)-39 | 0,025 | 16,2 |
| 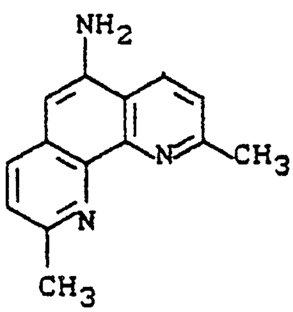 (I)-4 | 0,025 | 40,3 |

Tabelle D - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 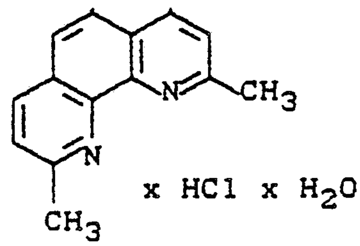 x HCl x $H_2O$ (I)-40 | 0,025 | 8,7 |

Tabelle D - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 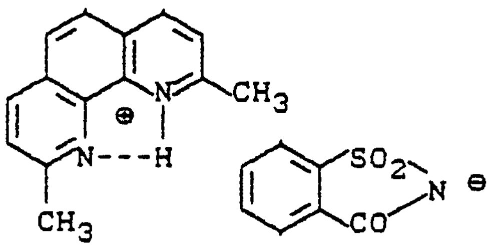 (I)-53 | 0,025 | 12,5 |
| 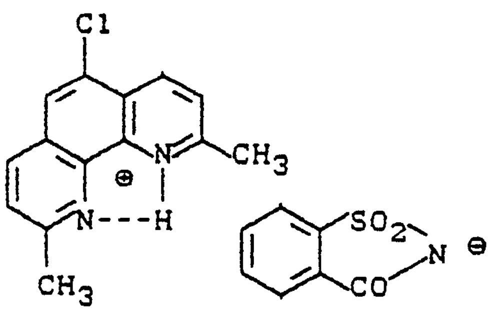 (I)-7 | 0,025 | 12,5 |
| 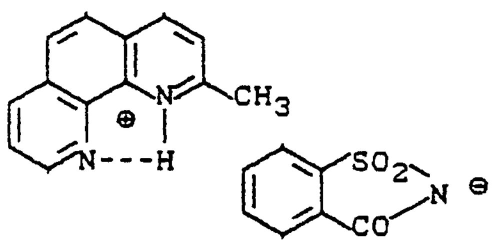 (I)-55 | 0,025 | 25 |

Tabelle D - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 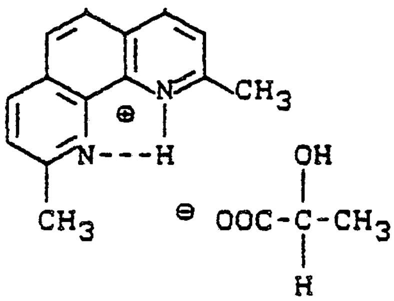 (I)-57 | 0,025 | 0 |
| 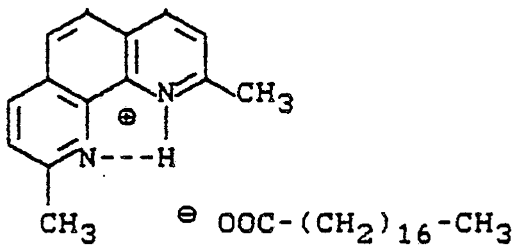 (I)-59 | 0,025 | 8,7 |
| 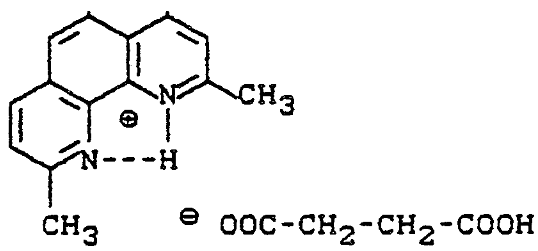 (I)-60 | 0,025 | 0 |

Beispiel E

Drechslera graminea-Test (Gerste) / Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 ° C aus. Dabei wird die Keimung der Gerste und gegebenenfalls auch der Pilzsporen eingeleitet. Anschließend sät man die vorgekeimte Gerste mit 2 × 50 Korn 3 cm tief in eine Standarderde und kultiviert sie im Gewächshaus bei einer Temperatur von ca. 18° C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

In diesem Test zeigen z.B. die erfindungsgemäßen Stoffe [(I)-17], [(I)-18] und [(I)-40] eine bessere Wirksamkeit als die Vergleichsubstanz (A).

## Tabelle E

Drechslera graminea-Test (Gerste) / Saatgutbehandlung
(syn. Helminthosporium gramineum)

| Wirkstoff | Wirkstoffaufwandmenge in mg/kg Saatgut | kranke Pflanzen in % der insgesamt aufgelaufenen Pflanzen |
|---|---|---|
| ungebeizt | - | 23,4 |
| 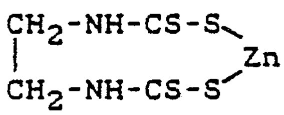 (bekannt) (A) | 500 | 18,0 |
| 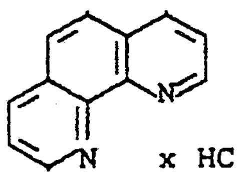 x HCl x $H_2O$ (I)-17 | 500 | 0,0 |
| 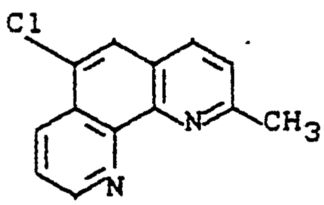 (I)-18 | 500 | 0,0 |
| 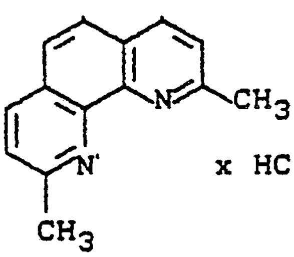 x HCl x $H_2O$ (I)-40 | 500 | 0,0 |

Beispiel F

Botrytis-Test (Bohne)/protektiv Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichsteil Wirkstoff mit den angegebenen Mengen Lösungsmittel und Emulgator und verdünnt das Konzentrat mit Wasser auf die gewünschte Konzentration.

Zuf Prüfung auf protektive Wirksamkeit bespritzt man junge Pflanzen mit der Wirkstoffzubereitung bis zur Tropfnäasse. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

In diesem Test zeigen z.B. die erfindungsgemäßen Stoffe [(I)-2], [(I)-30] und [(I)-35] eine bessere Wirksamkeit als die Vergleichssubstanz (B).

## Tabelle F

Botrytis-Test (Bohne)/protektiv

| Wirkstoff | Befall in % bei einer Wirkstoffkonzentration von 100 ppm |
|---|---|
| 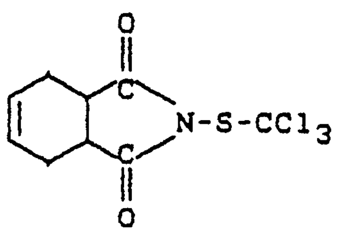 (bekannt) (B) | 38 |
| 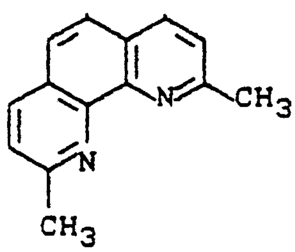 (I)-2 | 3 |
| 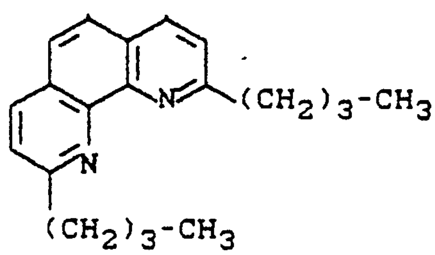 (I)-30 | 4 |

Tabelle F - Fortsetzung

Botrytis-Test (Bohne)/protektiv

| Wirkstoff | Befall in % bei einer Wirkstoffkonzentration von 100 ppm |
|---|---|
| 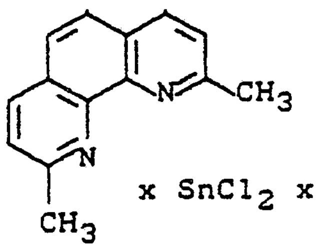 x $SnCl_2$ x $H_2O$ (I)-35 | 6 |

Beispiel G

Venturia-Test (Apfel) /protektiv/ Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigen z.B. die erfindungsgemäßen Stoffe [(I)-7], [(I)-53], [(I)-54], [(I)-56] und [(I)57] eine bessere Wirksamkeit als die Vergleichsubstanz (C).

## Tabelle G

Venturia-Test (Apfel) / protektiv

| Wirkstoff | Befall in % bei einer Wirkstoffkonzentration von 10 ppm |
|---|---|
| 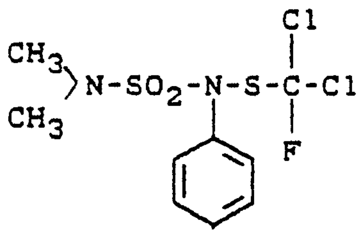 (bekannt) (C) | 26 |
| 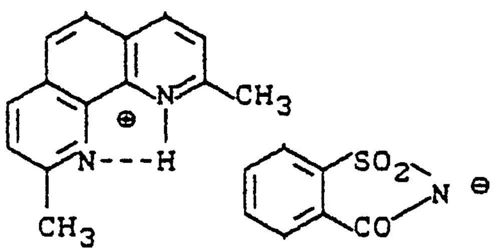 (I)-53 | 6 |
| 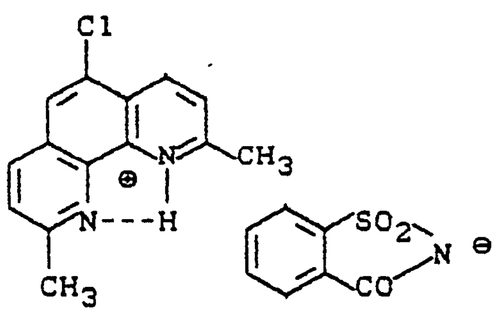 (I)-7 | 10 |

Tabelle G

Venturia-Test (Apfel) / protektiv/

| Wirkstoff | Befall in % bei einer Wirkstoffkonzentration von 10 ppm |
|---|---|
| 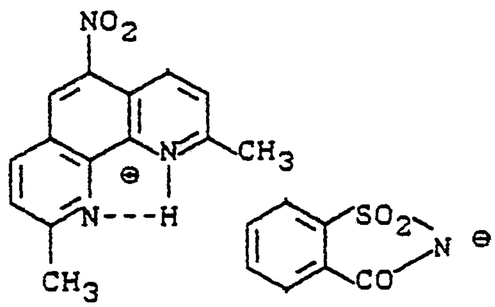 (I)-54 | 9 |
| 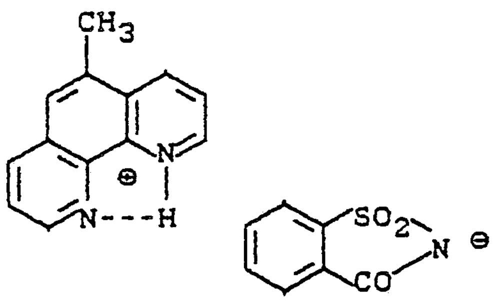 (I)-56 | 13 |
| 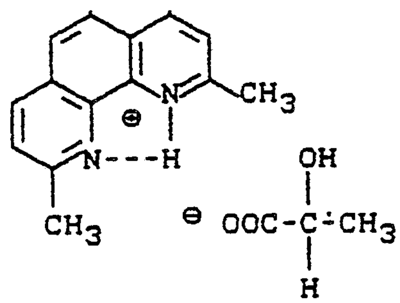 (I)-57 | 11 |

**Ansprüche**

1. Schädlingsbekämpfungsmittel, gekennzeichnet durch einen Gehalt an mindestens einem substituierten 1,10-Phenanthrolin der allgemeinen Formel (I)

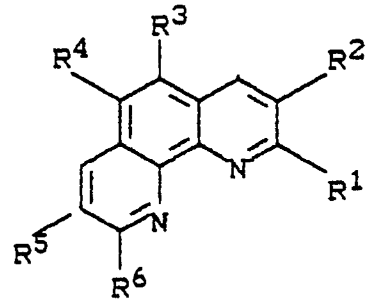

(I)

in welcher

$R^1$ und $R^6$ gleich oder verschieden sind und für Wasserstoff, Alkyl, Halogenalkyl, Halogen, Alkoxy, Alkylthio, Alkylamino oder Dialkylamino stehen,

$R^2$ und $R^5$ gleich oder verschieden sind und für Wasserstoff oder Alkyl stehen,

$R^3$ und $R^4$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, gegebenenfalls substituiertes Aryl, Amino, Alkylamino, gegebenenfalls substituiertes Arylamino, Aralkylamino, Dialkylamino, Diaralkylamino oder für die Gruppierungen $-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen,

worin

$R^7$, $R^8$, $R^9$, $R^{10}$, $R^{11}$, $R^{12}$ und $R^{13}$ gleich oder verschieden sind und für Alkyl oder gegebenenfalls substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe $N-R^{14}$ stehen, worin $R^{14}$ für Wasserstoff oder Alkyl steht,

sowie deren Säureadditions-Salze und Metallsalz-Komplexe.

2. Schädlingsbekämpfungsmittel gemäß Anspruch 1, worin in der Formel (I)

$R^1$ und $R^6$ gleich oder verschieden sind und für Wasserstoff, geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Alkoxy oder Alkylthio mit jeweils 1 bis 4 Kohlenstoffatomen, geradkettiges oder verzweigtes Alkylamino oder Dialkylamino mit jeweils 1 bis 4 Kohlenstoffatomen je Alkylrest stehen, und

$R^2$ und $R^5$ gleich oder verschieden sind und für Wasserstoff oder geradkettiges oder verzweigtes Alkyl mit 1 bis 4 Kohlenstoffatomen stehen.

$R^3$ und $R^4$ gleich oder verschieden sind und für Wasserstoff, für geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Hydroxy, Alkoxy oder Alkylthio mit jeweils 1 bis 4 Kohlenstoffatomen, Mercapto, Alkylsulfinyl oder Alkylsulfonyl mit jeweils 1 bis 4 Kohlenstoffatomen, Nitro, gegebenenfalls einfach oder zweifach, gleich oder verschieden durch Halogen, Alkyl oder Halogenalkyl mit jeweils 1 bis 3 Kohlenstoffatomen und 1 bis 5 gleichen oder verschiedenen Halogenatomen substituiertes Phenyl, ferner für Amino, für jeweils geradkettiges oder verzweigtes Alkylamino oder Dialkylamino mit jeweils 1 bis 4 Kohlenstoffatomen je Alkylteil, gegebenenfalls einfach oder zweifach,
gleich oder verschieden durch Halogen, Alkyl oder Halogenalkyl mit jeweils 1 bis 3 Kohlenstoffatomen und gegebenenfalls 1 bis 5 gleichen oder verschiedenen Halogenatomen substituiertes Phenylamino, für jeweils gegebenenfalls einfach bis mehrfach, gleich oder verschieden durch Halogen substituiertes Phenylalkylamino oder Diphenylalkylamino mit jeweils 1 oder 2 Kohlenstoffatomen im Alkylteil oder für die Gurppierungen
$-NH-CO-R^7$, $-NH-CY-X-R^8$, $-NH-SO_2-R^9$, $,Y^1-CO-R^{10}$, $-Y^2-CO-X^1-R^{11}$, $-Y^3-CS-X^2-R^{12}$ oder $-CO-R^{13}$ stehen, worin

$R^7$, $R^8$, $R^9$, $R^{10}$, $R^{11}$, $R^{12}$ und $R^{13}$ gleich oder verschieden sind und für geradkettiges oder verzweigtes Alkyl mit 1 bis 4 Kohlenstoffatomen oder für gegebenenfalls einfach bis dreifach, gleich oder verschieden durch Halogen, Nitro oder Alkyl mit 1 bis 4 Kohlenstoffatomen substituiertes Phenyl stehen,

Y, $Y^1$, $Y^2$ und $Y^3$ gleich oder verschieden sind und für Sauerstoff oder Schwefel stehen und,

X, $X^1$ und $X^2$ gleich oder verschieden sind und für Sauerstoff, Schwefel oder für die Gruppe $N-R^{14}$

stehen, worin $R^{14}$ für Wasserstoff oder geradkettiges oder verzweigtes Alkyl mit 1 bis 4 Kohlenstoffatomen steht,

sowie deren Säureadditionssalze und Metallsalz-Komplexe.

3. Schädlingsbekämpfungsmittel gemäß Anspruch 1, worin in der Formel (I)

$R^1$ und $R^6$ gleich oder verschieden sind und für Wasserstoff, Methyl, Ethyl, n-und i-Propyl, n-, i-, s-und t-Butyl, Chlormethyl, Brommethyl, Trichlormethyl, Chlor, Brom, Methoxy, Ethoxy, Methylamino oder Dimethylamino stehen,

$R^2$ und $R^5$ gleich oder verschieden sind und für Wasserstoff, Methyl oder Ethyl stehen,

$R^3$ und $R^4$ gleich oder verscheieden sind und für Wasserstoff, Methyl, Ethyl, n-und i-Propyl, Chlor, Brom, Iod, Hydroxy, Methoxy, Ethoxy, n-Propoxy, i-Propoxy, Mercapto, Methylthio, Ethylthio, n-Butylsulfinyl, Methylsulfonyl, Nitro, gegebenenfalls einfach oder zweifach, gleich oder verschieden durch Fluor, Chlor, Brom, Methyl, Ethyl, i-Propyl oder Trifluormethyl substituiertes Phenyl, ferner für Amino, Methylamino, Ethylamino, 2,6-Dichlorbenzylamino, Dimethylamino, Diethylamino, gegebenenfalls einfach oder zweifach, gleich oder verschieden durch Fluor, Chlor, Brom, Methyl, Ethyl, i-Propyl oder Trifluormethyl substituiertes Phenylamino oder für die Gruppierungen $-NH-CO-R^7$, $-NH-CY-X-R^8$, $-NH-SO_2R^9$, $-Y^1-CO-R^{10}$, $-Y^2-CO-X^1-R^{11}$, $-Y^3-CS-X^2-R^{12}$ oder $-CO-R^{13}$ stehen,

worin

$R^7$, $R^8$, $R^9$, $R^{10}$, $R^{11}$, $R^{12}$ und $R^{13}$ gleich oder verschieden sind und für Methyl, Ethyl, n-und i-Propyl, n-, i-s-und t-Butyl oder für gegebenenfalls einfach oder zweifach, gleich oder verschieden durch Fluor, Chlor, Brom, Nitro, Methyl, Ethyl, Methoxy oder Trifluormethyl substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe $N-R^{14}$ stehen, worin

$R^{14}$ für Wasserstoff oder Methyl steht, deren Säureadditions-Salze und Metallsalz-Komplexe.

4. Verwendung von substituierten 1,10-Phenanthrolinen der Formel (I) gemäß Anspruch 1 zur Bekämpfung von Schädlingen.

5. Verfahren zur Bekämpfung von Schädlingen, dadurch gekennzeichnet, daß man substituierte 1,10-Phenanthroline der Formel (I) gemäß Anspruch 1 auf Schädlinge und/oder ihren Lebensraum einwirken läßt.

6. Verfahren zur Herstellung von Schäklingsbekämpfungsmitteln, dadurch gekennzeichnet, daß man substituierte 1,10-Phenanthroline der Formel (I) gemäß Anspruch 1 mit Streckmitteln und/oder oberflächenaktiven Stoffen vermischt.

7. Substituierte 1,10-Phenanthroline der Formel (Ia),

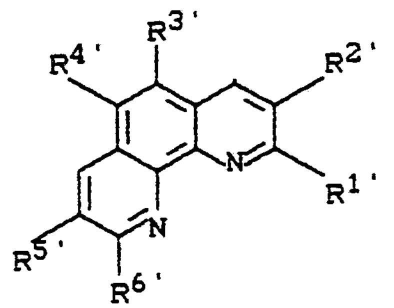

(Ia)

in welcher

$R^{1'}$ und $R^{6'}$ gleich oder verschieden sind und für Wasserstoff, Alkyl, Halogenalkyl, Halogen, Alkoxy, Alkythio, Alkylamino oder Dialkylamino stehen,

$R^{2'}$ und $R^{5'}$ gleich oder verschieden sind und für Wasserstoff oder Alkyl stehen,

$R^{3'}$ und $R^{4'}$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, gegebenenfalls substituiertes Aryl, Amino, Alkylamino, gegebenenfalls jeweils substituiertes Arylamino oder Aralkylamino, Dialkylamino, gegebenenfalls im Aryteil substituiertes Diaralkylamino oder für die Gruppierungen

$-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen,

worin

$R^7$, $R^8$, $R^9$, $R^{10}$, $R^{11}$, $R^{12}$ und $R^{13}$ gleich oder verschieden sind und für Alkyl oder gegebenenfalls substituiertes Phenyl stehen und

Y, $Y^1$, $Y^2$ und $Y^3$ gleich oder verschieden sind und für Sauerstoff oder Schwefel stehen, und

X, $X^1$ und $X^2$ gleich oder verschieden sind und für Sauerstoff, Schwefel oder für die Gruppe N-$R^{14}$ stehen, worin $R^{14}$ für Wasserstoff oder Alkyl steht,

deren Säureadditionssalze und Metallsalz-Komplexe,

wobei jedoch a) $R^{1'}$ und $R^{6'}$ nicht gleichzeitig für Wasserstoff stehen oder b) $R^{2'}$, $R^{3'}$, $R^{4'}$und $R^{5'}$ nicht gleichzeitig für Wasserstoff stehen oder c) für den Fall, daß $R^{3'}$, für Wasserstoff oder Alkyl steht, $R^{1'}$ nicht gleichzeitig für Methyl steht.

8. Substituierte 1,10-Phenanthroline gemäß Anspruch 7, worin in der Formel (Ia)

$R^{1'}$ und $R^{6'}$ gleich oder verschieden sind und für Wasserstoff, geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Alkoxy oder Alkylthio mit jeweils 1 bis 4 Kohlenstoffatomen, geradkettiges oder verzweigtes Alkylamino oder Dialkylamino mit jeweils 1 bis 4 Kohlenstoffatomen je Alkylrest stehen,

$R^{2'}$ und $R^{5'}$ gleich oder verschieden sind und für Wasserstoff oder geradkettiges oder verzweigtes Alkyl mit 1 bis 4 Kohlenstoffatomen stehen,

$R^{3'}$ und $R^{4'}$ gleich oder verschieden sind und für Wasserstoff, für geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Hydroxy, Alkoxy oder Alkylthio mit jeweils 1 bis 4 Kohlenstoffatomen, Mercapto, Alkylsulfinyl oder Alkylsulfonyl mit jeweils 1 bis 4 Kohlenstoffatomen, Nitro, gegebenenfalls einfach oder zweifach, gleich oder verschieden durch Halogen, Alkyl oder Halogenalkyl mit jeweils 1 bis 3 Kohlenstoffatomen und gegebenenfalls 1 bis 5 gleichen oder verschiedenen Halogenatomen substituiertes Phenyl, ferner für Amino, für jeweils geradkettiges oder verzweigtes Alkylamino oder Dialkylamino mit jeweils 1 bis 4 Kohlenstoffatomen je Alkylrest, gegenbenenfalls einfach oder zweifach, gleich oder verschieden durch Halogen, Alkyl oder Halogenalkyl mit jeweils 1 bis 3 Kohlenstoffatomen, und gegebenenfalls 1 bis 5 gleichen oder verschiedenen Halogenatomen substituiertes Phenylamino, für jeweils gegebenfalls einfach bis mehrfach, gleich oder verschieden durch Halogen substituiertes Phenylalkylamino oder Diphenylalkylamino mit jeweils 1 oder 2 Kohlenstoffatomen im Alkylteil oder für die Gruppierungen

$-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen, worin

$R^7$, $R^8$, $R^9$, $R^{10}$, $R^{11}$, $R^{12}$ und $R^{13}$ gleich oder verschieden sind und für geradkettiges oder verzweigtes Alkyl mit 1 bis 4 Kohlenstoffatomen oder für gegebenenfalls einfach bis dreifach, gleich oder verschieden durch Halogen, Nitro oder Alkyl mit 1 bis 4 Kohlenstoffatomen substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe N-$R^{14}$ stehen,

worin $R^{14}$ für Wasserstoff oder geradkettiges oder verzweigtes Alkyl mit 1 bis 4 Kohlenstoffatomen steht,

deren Säureadditionssalze und Metallsalz-Komplexe,

wobei jedoch a) $R^{1'}$ und $R^{6'}$ nicht gleichzeitig für Wasserstoff stehen oder b) $R^{2'}$, $R^{3'}$, $R^{4'}$ und $R^{5'}$ nicht gelichzeitig für Wasserstoff stehen, sowie c) für den Fall, daß $R^{3'}$ für Wasserstoff oder Alkyl steht, $R^{1'}$ nicht gleichzeitig für Methyl steht.

9. Substituierte 1,10-Phenanthroline gemäß Anspruch 1, worin in der Formel (Ia)

$R^{1'}$ und $R^{6'}$, gleich oder verschieden sind und für Wasserstoff, Methyl, Ethyl, n-und i-Propyl oder n-, i-, s-oder t-butyl stehen,

$R^{2'}$ und $R^{5'}$ gleich oder verschieden sind und für Wasserstoff, Methyl oder Ethyl stehen,

$R^{3'}$ und $R^{4'}$ gleich oder verschieden sind und für Wasserstoff, Methyl, Chlor, Methoxy, Nitro, Amino, 2,6-Dichlorbenzylamino, Ethylcarbonylamino

oder für den Rest

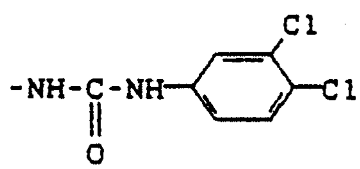

steht,

deren Säureadditions-Salze und Metallsalz-Komplexe,

wobei jedoch a) $R^{1'}$ und $R^{6'}$ nicht gleichzeitig für Wasserstoff stehen oder b) $R^{2'}$, $R^{3'}$, $R^{4'}$ und $R^{5'}$nicht gleichzeitig für Wasserstoff stehen, sowie c) für den Fall, daß $R^{3'}$ für Wasserstoff oder Alkyl steht, $R^{1'}$ nicht gleichzeitig für Methyl steht.

10. Verfahren zur Herstellung von substituierten 1,10-Phenanthrolinen der Formel (Ia),

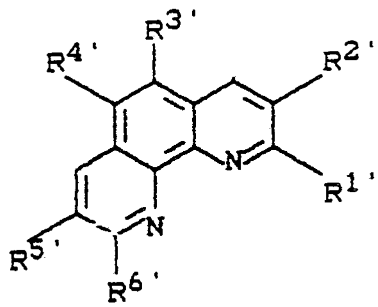

(Ia)

in welcher

$R^{1'}$ und $R^{6'}$ gleich oder verschieden sind und für Wasserstoff, Alkyl, Halogenalkyl, Halogen, Alkoxy, Alkylthio, Alkylamino oder Dialkylamino stehen,

$R^{2'}$ und $R^{5'}$ gleich oder verschieden sind und für Wasserstoff oder Alkyl stehen,

$R^{3'}$ und $R^{4'}$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, gegebenenfalls substituiertes Aryl, Amino, Alkylamino, gegebenenfalls jeweils substituiertes Arylamino oder Aralkylamino, Dialkylamino, gegebenenfalls im Arylteil substituiertes Diaralkylamino oder für die Gruppierungen

$-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen, worin

$R^7$, $R^8$, $R^9$, $R^{10}$, $R^{11}$, $R^{12}$ und $R^{13}$ gleich oder verschieden sind und für Alkyl oder gegebenenfalls substituiertes Phenyl stehen,

Y, $Y^1$, $Y^2$ und $Y^3$ gleich oder verschieden sind und für Sauerstoff oder Schwefel stehen,

X, $X^1$ und $X^2$ gleich oder verschieden sind und für Sauerstoff, Schwefel oder für die Gruppe $N-R^{14}$ stehen,

worin $R^{14}$ für Wasserstoff oder Alkyl steht,

deren Säureadditionssalze und Metallsalz-Komplexe, wobei jedoch a) $R^{1'}$ und $R^{6'}$ nicht gleichzeitig für Wasserstoff stehen oder b) $R^{2'}$, $R^{3'}$, $R^{4'}$ und $R^{5'}$ nicht gleichzeitig für Wasserstoff stehen oder c) für den Fall, daß $R^{3'}$ für Wasserstoff oder oder Alkyl steht, $R^{1'}$ nicht gleichzeitig für Methyl steht,

dadurch gekennzeichnet, daß man

A) 8-Aminochinoline der Formel (II)

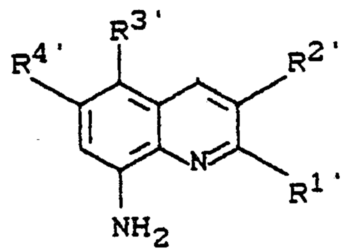

(II)

in welcher

$R^{1'}$, $R^{2'}$, $R^{3'}$ und $R^{4'}$ die oben angegebene Bedeutung haben, wobei jedoch für den Fall, daß $R^{3'}$ für Wasserstoff oder Alkyl steht, $R^{1'}$ nicht gleichzeitig für Methyl steht,

α) mit Aldehyden der Formel (III)

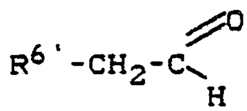

(III)

in welcher

$R^{6'}$ die oben angegebene Bedeutung hat,

gegebenfalls in Gegenwart von Verdünnungsmitteln sowie gegebenenfalls in Gegenwart starker Säuren cylisiert und die so erhaltenen Produkte gegebenenfalls in Säureadditions-Salze und Metallsalz-Komplexe überführt, oder

β) mit α,β-ungesättigten Aldehyden der Formel (IIIa),

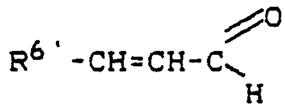

(IIIa)

in welcher

$R^{6'}$ die oben angegebene Bedeutung hat,

gegebenenfalls in Gegenwart von Verdünnungsmitteln sowie gegebenenfalls in Gegenwart einer starken Säure oder gegebenfalls in Gegenwart eines Oxidationsmittels oder unter Wasserabscheidung gegebenenfalls in Gegenwart eines Katalysators cyclisiert und die so erhaltenen Produkte gegebenenfalls in Säureadditions-Salze und Metallsalz-Komplexe überführt,

oder daß man die noch nicht bekannten substituierten 1,10-Phenanthroline der Formel (Ia) erhält, wenn man

B) substituierte 1,10-Phenanthrolin-Derivate der Formel (Ib),

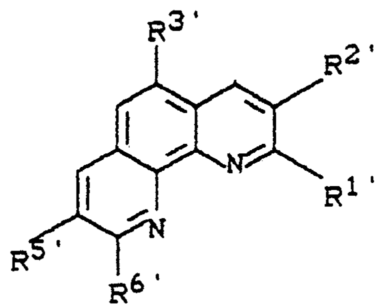

(Ib)

in welcher

$R^{1'}$, $R^{2'}$, $R^{3'}$, $R^{5'}$ und $R^{6'}$ die oben angegebene Bedeutung haben, wobei jedoch a) $R^{1'}$ und $R^{6'}$ nicht gleichzeitg für Wasserstoff stehen oder b) für den Fall, daß $R^{3'}$ für Wasserstoff oder Alkyl steht, $R^{1'}$ nicht gleichzeitig für Methyl steht,

zunächst in einer 1, Stufe in allgemein üblicher Art und Weise mit elektrophilen Agenzien der Formel (IV),

$R^{15}$-E (IV)

in welcher

$R^{15}$ für Halogen, Nitro, Hydroxysulfonyl, Chlorsulfonyl, Alkyl, Formyl, Alkanoyl oder Aroyl steht und

E für eine elektronenanziehende Abgangsgruppierung steht,

oder mit anderen üblichen elektrophilen Reagenzien gegebenenfalls in Gegenwart eines Verdünnungsmittels und gegebenenfalls in Gegenwart eines Katalysators oder Reaktionshilfsmittels in 5- oder 6-Stellung substituiert und diese für den Fall, daß $R^{4'}$ in der Formel (Ia) von einer Nitro-in eine Aminofunktion überführt werden soll, in einer zweiten Stufe gegebenenfalls in Gegenwart von Verdünnungsmitteln und gegebenenfalls in Gegenwart eines Katalysators oder Reaktionshilfsmittels reduziert und gegebenenfalls die so erhaltenen substituierten 1,10-Phenanthroline in deren Säureadditions-Salze oder Metallsalz-Komplexe überführt,

oder daß man die noch nicht bekannten substituierten 1,10-Phenanthroline der Formel (Ia) erhält, wenn man

C) substituierte 1,10-Phenanthrolin-Derivate der Formel (Ic)

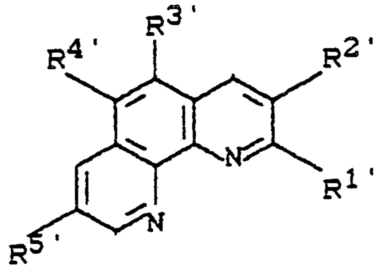

(Ic)

in welcher

$R^{1'}$, $R^{2'}$, $R^{3'}$, $R^{4'}$, und $R^{5'}$ die oben angegebene Bedeutung haben, wobei jedoch a) $R^{2'}$, $R^{3'}$, $R^{4'}$ und $R^{5'}$ nicht gleichzeitig für Wasserstoff stehen oder b) für den Fall, daß $R^{3'}$ für Wasserstoff oder Alkyl steht, $R^{1'}$ nicht gleichzeitig für Methyl steht,

in allgemein üblicher Art und Weise mit Lithiumverbindungen der Formel (V),

$R^{6'}$-Li (V)

in welcher

$R^{6'}$ die oben angegebene Bedeutung hat,

gegebenenfalls in Gegenwart von Verdünnungsmitteln und gegebenenfalls in Gegenwart eines Inertgases umsetzt, anschließend hydrolisiert, danach mit einem Oxidationsmittel dehydriert und die so erhaltenen Produkte gegebenenfalls in deren Säureadditions-Salze oder Metallsalz-Komplexe überführt,

oder

daß man die substituierten 1,10-Phenanthrolin-Derivate der Formel (Ia) erhält, wenn man

D) Verbindungen der Formel (Id),

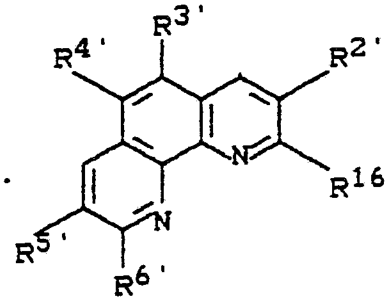

(Id)

in welcher

$R^{16}$ für Halogen steht und

$R^{2'}$, $R^{3'}$, $R^{4'}$, $R^{5'}$ und $R^{6'}$ die oben angegebene Bedeutung haben, wobei $R^{2'}$, $R^{3'}$, $R^{4'}$ und $R^{5'}$ nicht gleichzeitig für Wasserstoff stehen,

gegebenenfalls in Gegenwart von Verdünnungsmitteln und gegebenenfalls in Gegenwart eines Katalysators oder Reaktionshilfsmittels in 2-Stellung durch eine nucleophile Substitution mit Alkoholaten, Thiolaten oder N,N-Dialkylaminoverbindungen in die entsprechenden 2-Alkyloxy-, 2-Alkylthio-oder 2-N,N-Dialkylamino-1,10-Phenanthroline überführt und die so erhaltenen Produkte gegebenenfalls zu deren Säureadditions-Salzen und Metallsalz-Komplexen weiter umsetzt.